(19)

**Europäisches Patentamt**
**European Patent Office**
**Office européen des brevets**

(11)  **EP 3 339 861 B1**

(12)  # EUROPEAN PATENT SPECIFICATION

(45) Date of publication and mention
of the grant of the patent:
**27.09.2023  Bulletin 2023/39**

(21) Application number: **18154812.4**

(22) Date of filing: **13.06.2013**

(51) International Patent Classification (IPC):
**G01N 33/53** (2006.01)      **C12Q 1/00** (2006.01)
**G01N 33/68** (2006.01)

(52) Cooperative Patent Classification (CPC):
**G01N 33/689**; G01N 2800/368

(54) **BIOMARKER TEST FOR PREDICTION OR EARLY DETECTION OF PREECLAMPSIA**

BIOMARKERTEST ZUR VORHERSAGE ODER FRÜHERKENNUNG VON PRÄEKLAMPSIE

BIOMARQUEURS UTILISÉS POUR PRÉDIRE OU DÉTECTER PRÉCOCEMENT LA
PRÉ-ÉCLAMPSIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **15.06.2012  HU P1200368
10.09.2012  US 201261699193 P**

(43) Date of publication of application:
**27.06.2018  Bulletin 2018/26**

(62) Document number(s) of the earlier application(s) in
accordance with Art. 76 EPC:
**16186083.8 / 3 182 126
13803743.7 / 2 861 989**

(73) Proprietors:
• **Genesis Theranostix Korlatolt Felelossegu
Tarsasag
7624 Pécs (HU)**
• **Semmelweis University
1085 Budapest (HU)**
• **The United States of America, As Represented by
the Secretary, Dept. of Health & Human Services
Office of Technology Transfer
Bethesda, MD 20892-7660 (US)**

(72) Inventors:
• **THAN, Nandor
Grosse Pointe, MI 48236-1616 (US)**
• **TARCA, Adi, L.
Canton, MI 48188 (US)**
• **JUHÁSZ, Gábor
1117 Budapest (HU)**
• **KÉKESI, Adrienna, Katalin
1117 Budapest (HU)**
• **MEIRI, Hamutal
69121 Tel Aviv (IL)**
• **PAPP, Zoltán
1125 Budapest (HU)**
• **ROMERO, Roberto
Detroit, MI 48201 (US)**

(74) Representative: **Lengyel, Zsolt
Danubia
Patent & Law Office LLC
Bajcsy-Zsilinszky út 16
1051 Budapest (HU)**

(56) References cited:
**WO-A1-2009/108073      JP-A- 2008 241 704
US-A1- 2008 261 253**

• **LOCKWOOD C J; PAIDAS M J; LAPINSKI R;
TAYLOR R: "Elevated maternal plasma
beta-2-glycoprotein-1 (B2GP1) values precede
the clinical onset of preeclampsia", AMERICAN
JOURNAL OF OBSTETRICS AND
GYNECOLOGY1995, vol. 172, no. 1 part 2, 427,
1995, page 378, XP009504149,**
• **LIU C ET AL: "Proteomic analysis of human
serum for Finding pathogenic factors and
potential biomarkers in preeclampsia",
PLACENTA, W.B. SAUNDERS, GB, vol. 32, no. 2,
9 November 2010 (2010-11-09), pages 168-174,
XP028360520, ISSN: 0143-4004, DOI:
10.1016/J.PLACENTA.2010.11.007 [retrieved on
2010-11-17]**

EP 3 339 861 B1

- AUER J ET AL: "Serum profile in preeclampsia and intra-uterine growth restriction revealed by iTRAQ technology", JOURNAL OF PROTEOMICS, ELSEVIER, AMSTERDAM, NL, vol. 73, no. 5, 10 March 2010 (2010-03-10) , pages 1004-1017, XP026929961, ISSN: 1874-3919, DOI: 10.1016/J.JPROT.2009.12.014 [retrieved on 2010-01-14]
- SCHOLL P F ET AL: "Maternal serum proteome changes between the first and third trimester of pregnancy in rural Southern Nepal", PLACENTA, W.B. SAUNDERS, GB, vol. 33, no. 5, 8 February 2012 (2012-02-08), pages 424-432, XP028478163, ISSN: 0143-4004, DOI: 10.1016/J.PLACENTA.2012.02.009 [retrieved on 2012-02-15]
- T. VÁRKONYI ET AL: "Microarray Profiling Reveals That Placental Transcriptomes of Early-onset HELLP Syndrome and Preeclampsia Are Similar", PLACENTA, vol. 32, 1 February 2011 (2011-02-01), pages S21-S29, XP055217224, ISSN: 0143-4004, DOI: 10.1016/j.placenta.2010.04.014
- NANDOR GABOR THAN ET AL: "Integrated Systems Biology Approach Identifies Novel Maternal and Placental Pathways of Preeclampsia", FRONTIERS IN IMMUNOLOGY, vol. 9, 8 August 2018 (2018-08-08), XP055724102, DOI: 10.3389/fimmu.2018.01661
- TROND M. MICHELSEN ET AL: "The human placental proteome secreted into the maternal and fetal circulations in normal pregnancy based on 4-vessel sampling", THE FASEB JOURNAL, vol. 33, no. 2, 18 October 2018 (2018-10-18), pages 2944-2956, XP055725694, US ISSN: 0892-6638, DOI: 10.1096/fj.201801193R

Remarks:

The complete document including Reference Table(s) and the Sequence Listing(s) can be downloaded from the EPO website

**Description**

**FIELD OF THE DISCLOSURE**

The present disclosure provides a test which can be used to predict preeclampsia in pregnant women. More specifically, the disclosure provides a biomarker that can be used for early prediction and/or detection of preeclampsia.

**BACKGROUND OF THE DISCLOSURE**

Preeclampsia is a syndrome defined by pregnancy-induced hypertension and proteinuria, which can lead to eclampsia (convulsions), and other serious maternal and/or fetal complications. Preeclampsia is originated in early gestation from the failure of implantation mechanisms and/or placental development, and is thus closely related to complications of pregnancy in early gestation such as including but not limited to implantation failure, and threatened and spontaneous miscarriage, Preeclampsia affects approximately 5-7% of pregnant women (approximately 8,370,000 pregnant women worldwide per year) and is a major cause of maternal and perinatal mortality. Furthermore, women with preeclampsia have an 8-fold higher risk of cardiovascular death later in their life, and offspring born from pregnancies affected by preeclampsia have an increased risk of metabolic and cardiovascular disease and mortality later in life.

The present diagnostic criteria for preeclampsia set by the United States National High Blood Pressure Education Program Working Group on High Blood Pressure in Pregnancy include new-onset hypertension coupled with proteinuria that develops after 20 weeks of gestation in women with previously normal blood pressures. These criteria further define preeclampsia as systolic or diastolic blood pressures of $\geq$140 and/or $\geq$90 mmHg, respectively, measured at two or more different time points, at least 4 hours (h) but not more than 1 week apart, as well as proteinuria of $\geq$300 mg protein in a 24 h urine sample, or two random urine specimens obtained at least 4 h but not more than 1 week apart containing $\geq$1+ protein on a dipstick.

Based on the timing of the clinical manifestation, preeclampsia has been historically classified into different sub-forms, such as "term" ($\geq$37 weeks) and "preterm" (<37 weeks) or by using an alternative terminology "late-onset" and "early-onset" preeclampsia. The latter classification has not been uniformly used, but different studies have employed a range of gestational age cutoffs varying between 28 and 35 weeks for the distinction between early-onset and late-onset preeclampsia. Recently, it has been suggested to define 34 weeks as the gestational age cutoff between these two forms. It is important to note that preeclampsia may occur intrapartum or postpartum; thus, monitoring and evaluating the symptoms of preeclampsia should be continued during the postpartum period.

In 1954, it was first reported that preeclampsia may be associated with haemolysis, abnormal liver function and thrombocytopenia. Initially accepted to be a severe variant of preeclampsia, this group of symptoms later was suggested to constitute a separate clinical entity termed Haemolysis, Elevated Liver enzymes and Low Platelets (HELLP) syndrome. Supporting the idea that HELLP syndrome is a distinct condition, up to 20% of HELLP syndrome patients do not develop hypertension, 5-15% have minimal or no proteinuria and 15% show neither hypertension nor proteinuria. Moreover, laboratory findings in HELLP syndrome rarely correlate with the severity of hypertension or proteinuria.

In addition to the medical complications suffered by mothers and risks to the offspring, preeclampsia and HELLP syndrome cause approximately $7 billion in healthcare costs in the United States annually. Accordingly, there have been many attempts to provide a reliable predictive test for preeclampsia/HELLP syndrome. Previous attempts have involved assays for the concentrations of circulating biochemical markers in maternal blood but to date, the scientific literature on these approaches have been contradictory and inconclusive. There is a need in the art for new and improved methods of predicting and diagnosing these conditions.

**SUMMARY OF THE DISCLOSURE**

The present disclosure provides biomarker that allow for the prediction and/or early detection of preeclampsia.

More specifically, one embodiment includes a method for assessing, in the first trimester of a pregnancy, the risk of developing preterm severe preeclampsia before the 36th week in a woman, comprising:

determining the level of apolipoprotein H precursor, having Accession Number gi|153266841, in a blood sample obtained from the woman, wherein an increased level apolipoprotein H precursor is indicative of an increased risk of developing preterm severe preeclampsia before the 36th week.

Another embodiment includes the use of a kit in a method according to the invention comprising: detection mechanisms for determining level of apolipoprotein H precursor in a blood sample obtained from the woman; instructions how to (i) generate a dataset based on the determined level(s); (ii) assess the risk of developing preeclampsia in the woman; and (iii) determine a treatment regimen based on the assessed risk.

## BRIEF DESCRIPTION OF THE FIGURES

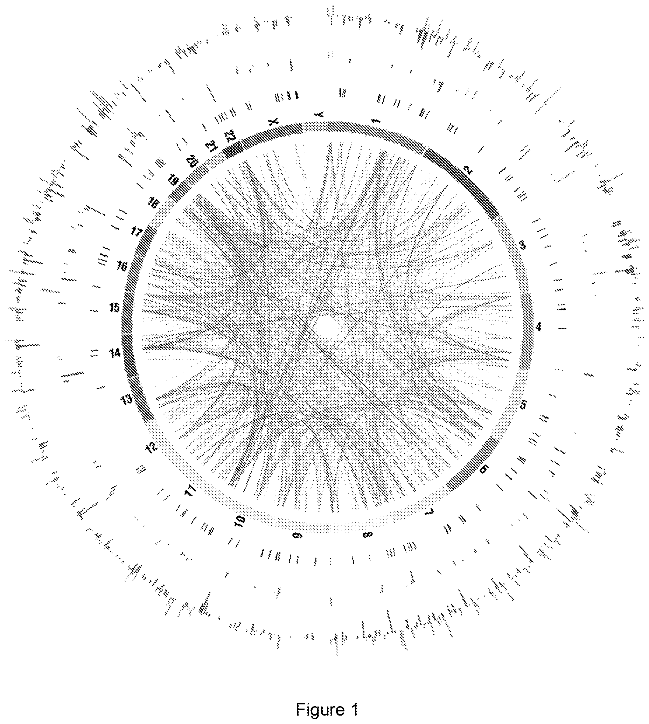

Figure 1. Figure 1 shows a genomic map of differentially expressed genes in preeclampsia. Circos visualization shows Chromosomes with solid lines in the inner circle. Curved lines connect the genomic coordinates of genes and transcription regulatory genes that are significantly correlated. Significance was determined by fitting a linear model between the expression level of gene and transcription regulatory gene pairs in all samples while controlling for FDR at 5%. Curves represent positive and negative correlations. The second circle shows the genomic location of genes with predominant placental expression (PPE) (black lines: non differentially expressed; grey lines: up- or down-regulated). The third and fourth circles show the locations of differentially expressed transcription regulatory genes and non-regulatory genes, respectively with inward-oriented bars (down-regulated) and outward-oriented bars (up-regulated). The height of the bars in the third and fourth circles represents the magnitude of gene expression changes.

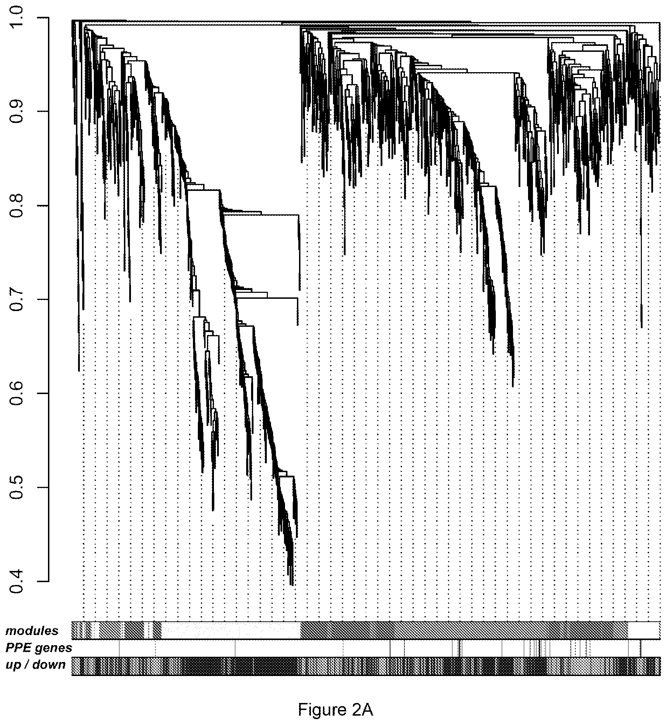

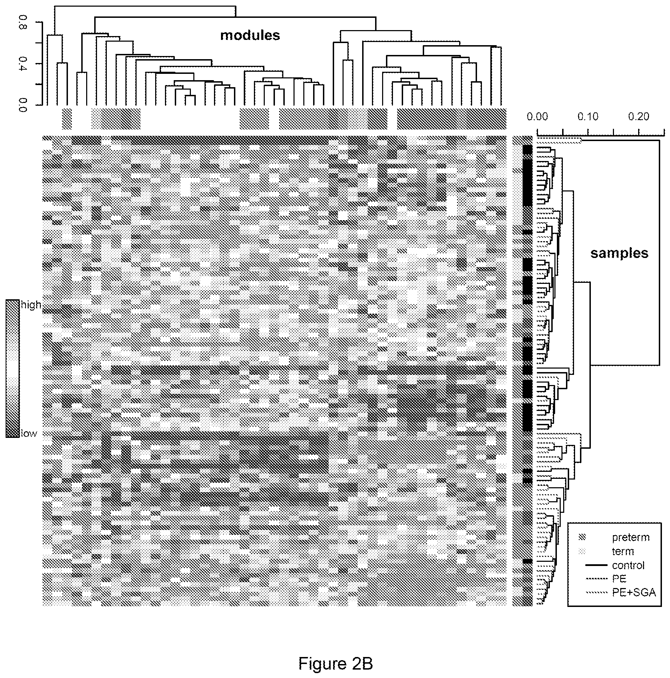

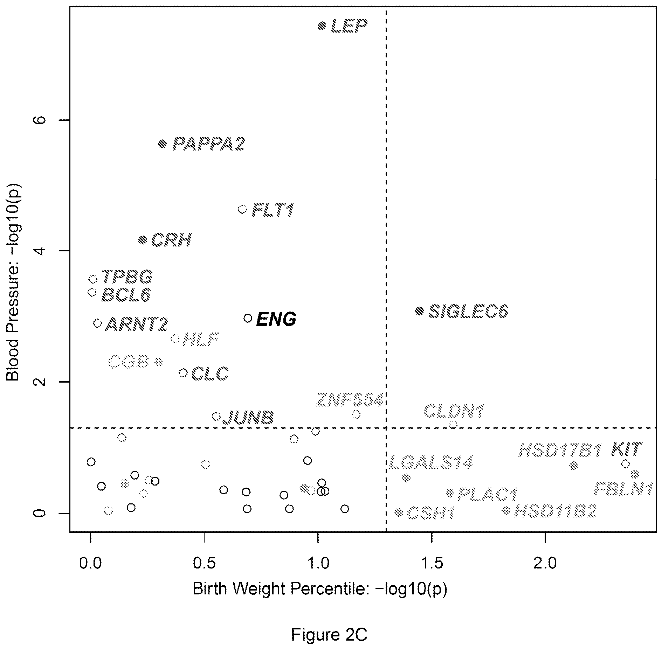

Figure 2. Figure 2A-2C shows that two gene modules in preeclampsia are enriched in PPE genes and are associated with mean arterial blood pressure and birth weight percentile. 2A) Gene modules identified from WGCNA analysis of microarray data. Dysregulated placental gene expression could be characterized by five gene modules within the 1,409 differentially expressed genes in preeclampsia, marked with different shades of grey. The height plotted on the y-axis represents the distance metric (1-TOM) used by WGCNA. Out of 38 PPE genes (black vertical lines), 33 belonged to the grey-scale modules (lighter grey; n=22 and darker grey; n=11). These two modules were also enriched in up-regulated and down-regulated genes marked under the modules with grey or black lines, respectively. 2B) Hierarchical clustering of qRT-PCR data obtained with 100 samples and selected 47 genes. Genes from light and dark grey modules clustered together in the validation sample-set. Importantly, 34 out of 60 samples from women with preeclampsia clustered tightly together. Pearson correlation was used for distance, and average for linkage. Samples (column leafs) were shaded according to patient groups and maturity status. 2C) Association of gene expression with mean arterial blood pressure and birth weight percentile. For each gene, a linear model was fitted (expression-blood pressure + birth weight percentile + gender + maturity status). The significance p-values ($-\log_{10}$ of) for the two coefficients (blood pressure and birth weight percentile) were plotted for all 47 genes. Genes were shaded according to module membership (except black color for those not differentially expressed on the microarray). Filled circles represent PPE genes, dashed lines the significance threshold at p=0.05. Note that 7 out of 9 genes related to birth weight percentile are from the light grey module, while 10 out of 15 genes related to blood pressure are from the dark grey module.

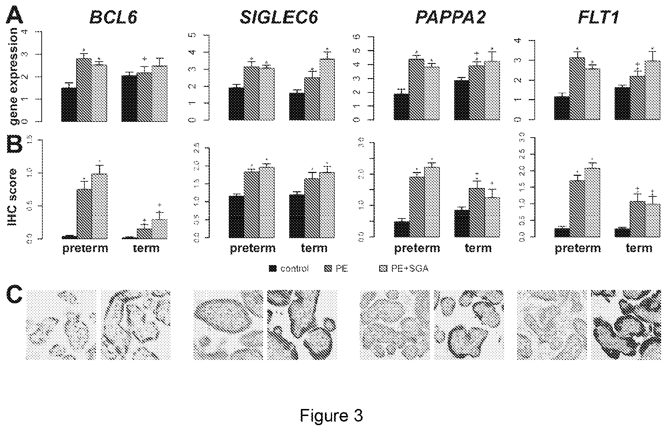

Figure 3. Figure 3A-3C shows that the expression of dark grey module genes changes in the same direction in preeclampsia subgroups. 4A-B) In each barplot, the left and right panels show significant differences ("*") in preterm and term preeclampsia samples, respectively. Gene expression 4A) and protein immunostainings 4B) show similar patterns in sub-groups of preeclampsia. When the change with preeclampsia in the preterm samples was significantly different than the change with preeclampsia in the term samples, a "+" sign indicates this interaction. Semiquantitative immunoscorings for four proteins 4B) validated gene expression data. 4C) Representative images from the four immunostainings. The same placenta from a preterm control (left, 29 weeks) and from a patient with preterm preeclampsia with SGA (right, 31 weeks) is shown for the four immunostainings (40x magnifications).

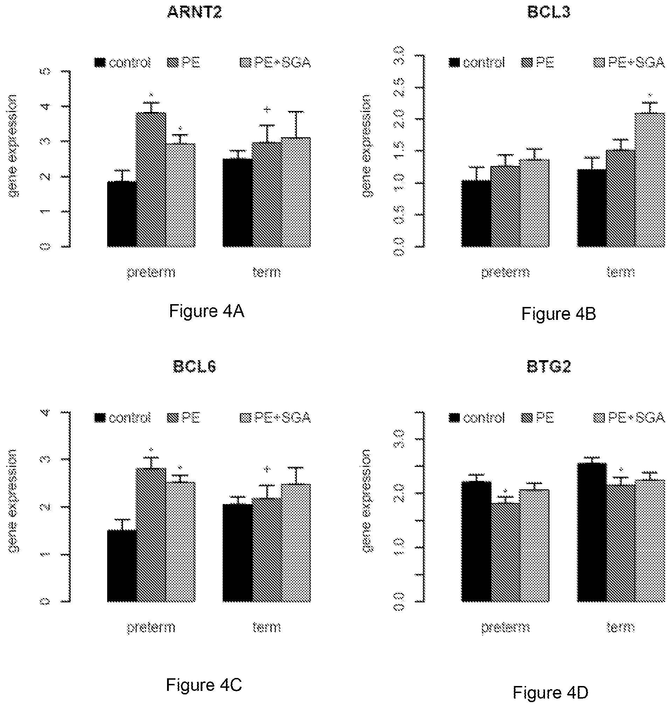

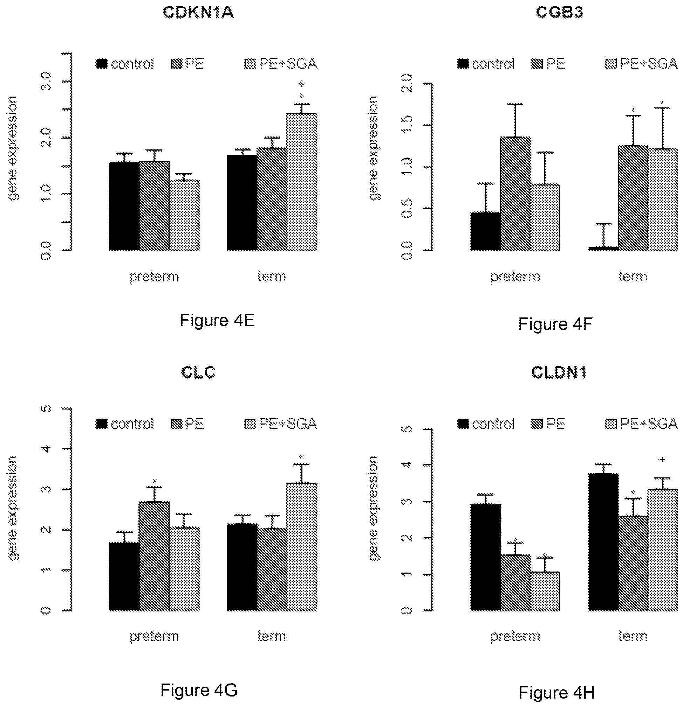

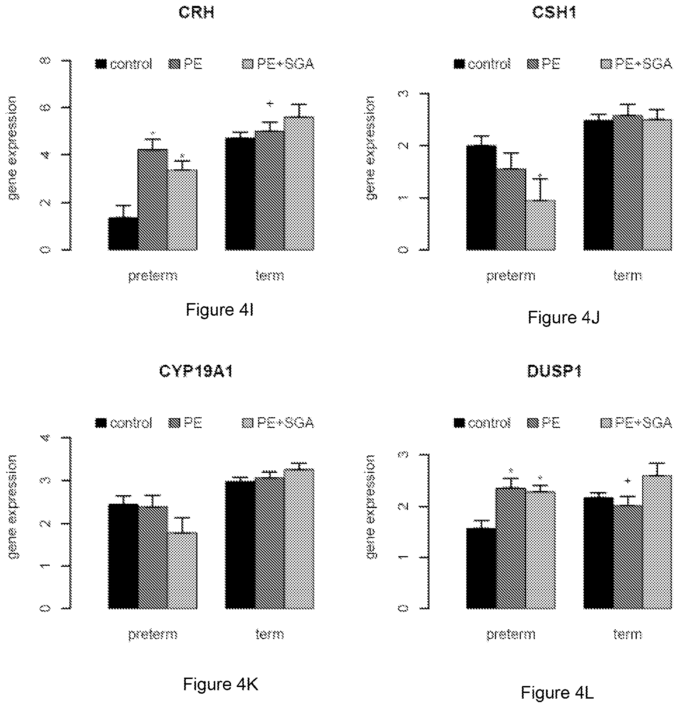

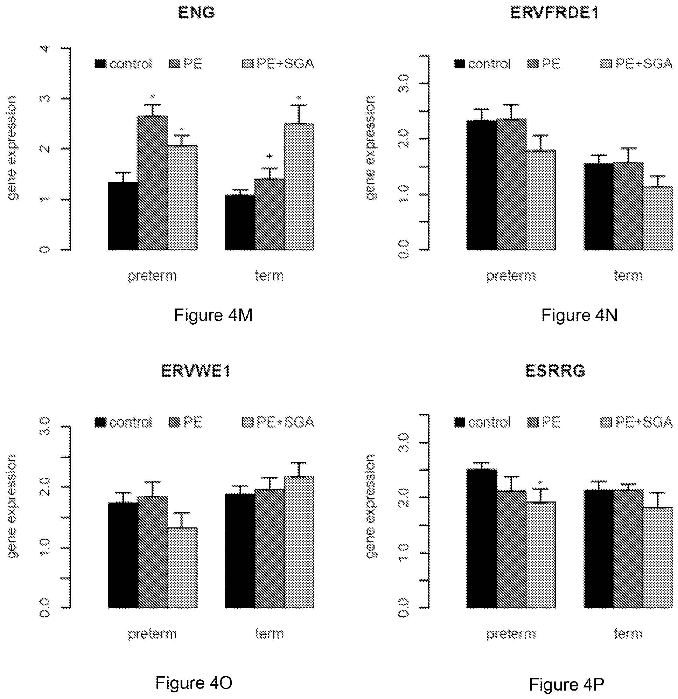

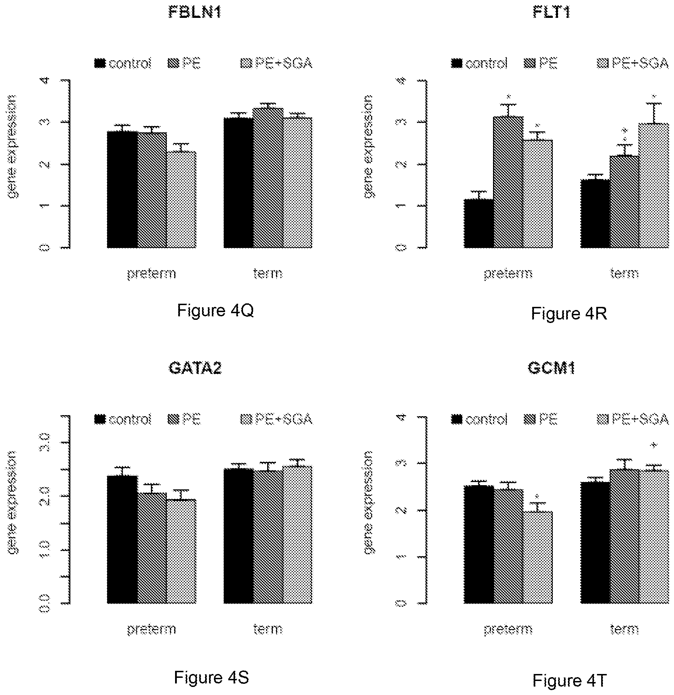

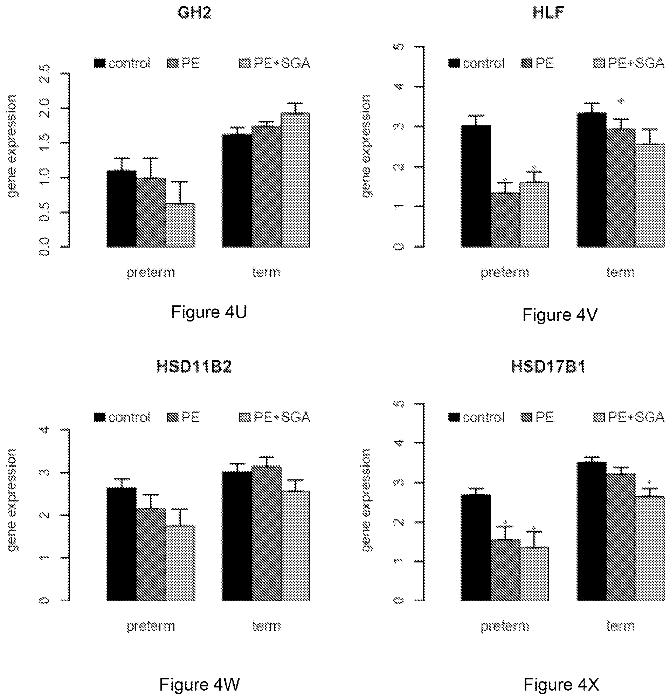

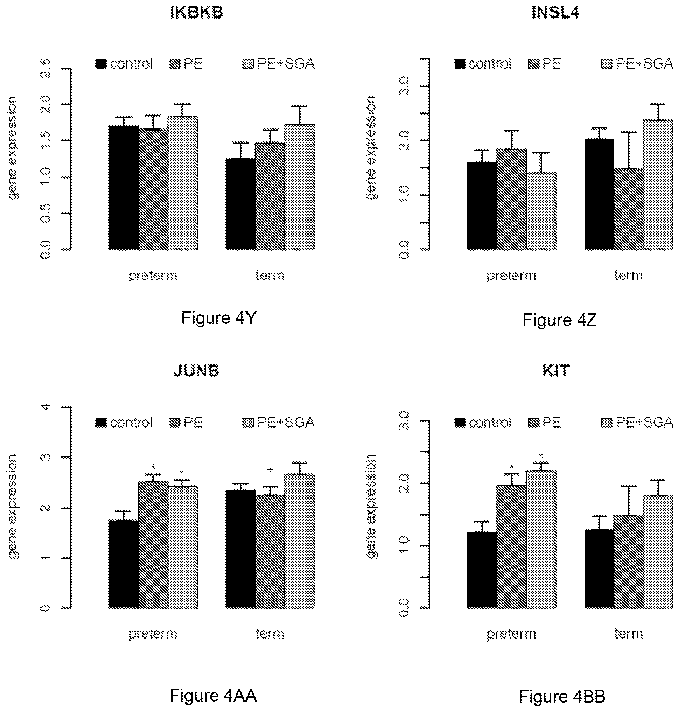

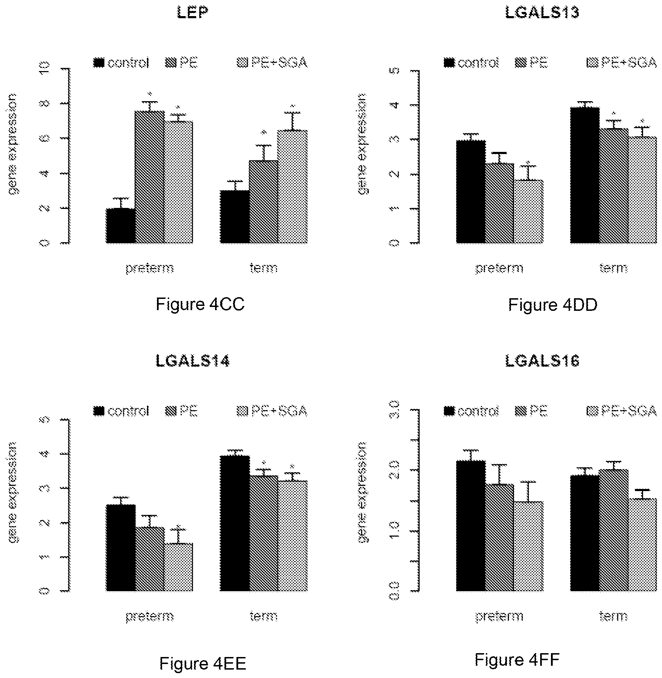

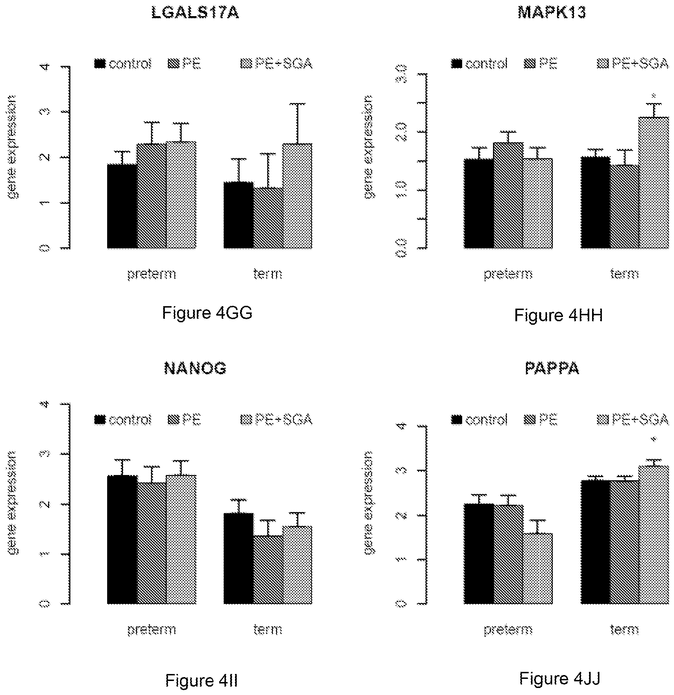

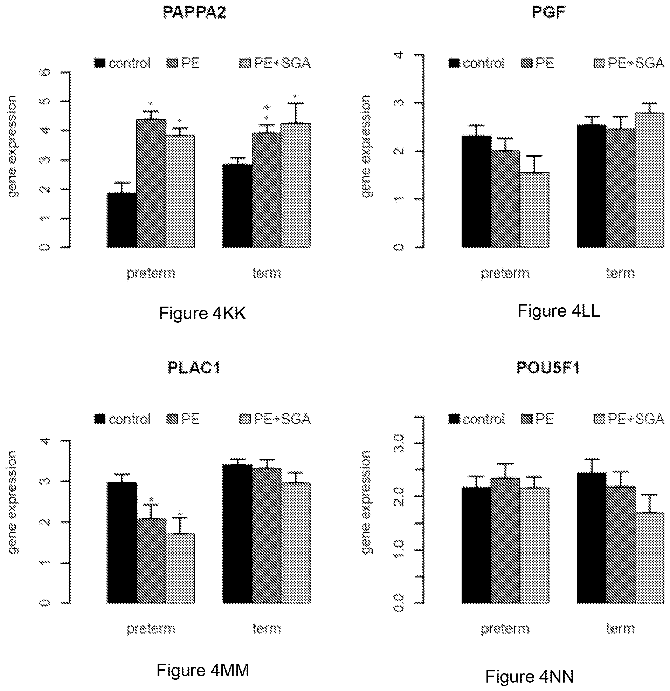

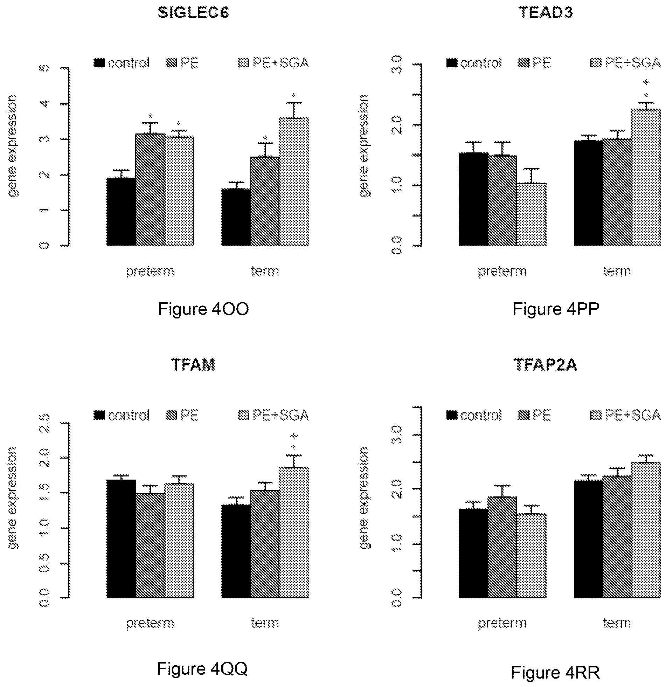

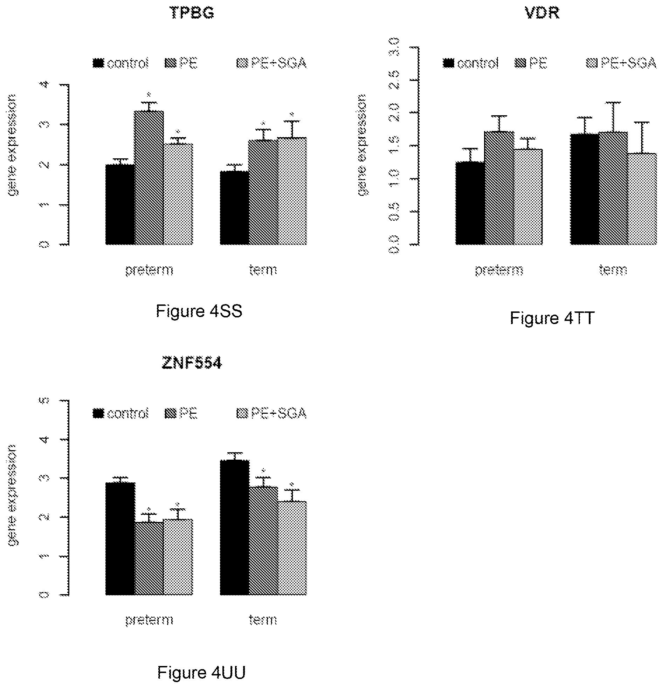

Figure 4. Figure 4A-4UU show gene comparisons for *ARNT2; BCL3; BCL6; BTG2; CDKN1A; CGB3; CLC; CLDN1; CRH; CSH1; CYP19A1; DUSP1; ENG; ERVFRDE1; ERVVVE1; ESRRG; FBLN1; FLT1; GATA2; GCM1; GH2; HLF; HSD11B2; HSD17B1; IKBKB; INSL4; JUNB; KIT; LEP; LGALS13; LGALS14; LGALS16; LGALS17A; MAPK13; NANOG; PAPPA; PAPPA2; PGF; PLAC1; POU5F1; SIGLEC6; TEAD3; TFAM; TFAP2A; TPBG; VDR;* and *ZNF554* respectively.

## DETAILED DESCRIPTION

As described above, there have been several attempts carried out over the past few years to develop and validate biomarkers for the early prediction of preeclampsia and/or HELLP syndrome; however, their results were not satisfactory. A possible reason for this is that the early diagnosis of syndromes with a heterogeneous molecular background cannot be solved with the utilization of only one or two biomarker molecules.

The present disclosure describes the use of a multidisciplinary systems biological approach which led to the identification of unique gene-, protein- and hormone biomarkers, which are repeatedly detectable in the 7-9th weeks of gestation. This aim of the present disclosure was achieved by the inclusion of several high-dimensional techniques: 1) whole genome transcriptomics of the placenta; 2) high-throughput qRT-PCR expressional profiling of the placenta; 3) high-throughput tissue microarray protein expression profiling of the placenta; 4) neural network analysis to select best combinations of candidate biomarkers to predict blood pressure and birth weight; 5) linear discriminant analysis model to provide sensitivity and specificity measures for preeclampsia prediction and 6) 2D-DIGE proteomics of maternal sera in early-pregnancy.

Whole-genome transcriptomics study of 17 placentas identified placenta- and pregnancy-specific genes differentially expressed in the placenta in preeclampsia and/or HELLP syndrome. The products of this set of genes can be identified in the maternal serum in large amounts in pregnancy, and thus, their expression and differential regulation is pregnancy-specific. However, their changes may not only be specific for preeclampsia, but also for other obstetrical syndromes.

High-throughput qRT-PCR expressional profiling of 100 placentas validated selected putative preeclampsia biomarkers at the RNA level.

High-throughput tissue microarray protein expression profiling of 100 placentas validated selected putative preeclampsia biomarkers at the protein level.

Neural network analysis supported the selection of the best combinations of putative preeclampsia biomarker genes, which expression can predict blood pressure and birth weight.

The Linear Discriminant Analysis showed that the average sensitivity and specificity of transcriptomic biomarkers for the detection of preeclampsia was 91.5% and 75%, respectively.

2D-DIGE proteomics of maternal sera in early-pregnancy revealed that the proteome of first trimester maternal blood in women with early-onset or late-onset preeclampsia differs from that of normal pregnant women, and these differences are partially different in the two subtypes of preeclampsia. Although these inflammatory and/or metabolic markers are not specific for pregnancy, they can differentiate between the two subtypes of preeclampsia. The combination of these transcriptomic and proteomic biomarker candidates resulted in a panel of molecules, which can detect preeclampsia-specific changes in maternal blood, and can also differentiate between the different subtypes of preeclampsia. A number of methods for obtaining expression data can be used singly or in combination for determining expression patterns and profiles in the context of the present disclosure. For example, DNA and RNA expression patterns can be evaluated by northern analysis, PCR, RT-PCR, quantitative real-time RT-PCR analysis with TaqMan assays, FRET detection, monitoring one or more molecular beacon, hybridization to an oligonucleotide array, hybridization to a cDNA array, hybridization to a polynucleotide array, hybridization to a liquid microarray, hybridization to a microelectric array, molecular beacons, cDNA sequencing, clone hybridization, cDNA fragment fingerprinting, serial analysis of gene expression (SAGE), subtractive hybridization, differential display and/or differential screening.

Gene expression changes can be related to epigenetic variations (e.g. DNA methylation). Epigenetic regulation mechanisms do not involve a change to the DNA sequence. Instead, epigenetic variations include covalent modification of DNA, RNA, and the proteins associated with DNA. These in turn can result in changes to the conformation of DNA and accessibility of regulators to the DNA. Such changes cannot be identified simply by gene sequencing. Janssen, B.G. et al., Particle and Fibre Toxicology, 10:22 (2013) studied methylation in placental tissue using methods published by Tabish, A.M. et al., PLoS ONE 2012, 7:e34674 and by Godderis, L. et al., Epigenomics 4:269-277 (2012). MS-MLPA (Methylation-specific Multiplex ligation-dependent probe amplification) can be used to study methylation status of specific genes, for example in Proctor, M. et al., Clin. Chem. 52:1276-1283 (2006). Materials and methods for MS-MLPA as used in published studies can be obtained from MRC-Holland, Amsterdam, The Netherlands. Additional methods are reviewed and compared in Shen, L. et al., Curr. Opin. Clin. Nutr. Metab. Care. 10:576-81 (2007); Gu H et al., Nature Methods 7:133-138 (2010); Bock C et al., Nature Biotech. 28:1106-1114 (2010); Harris RA et al., Nature Biotech. 28:1097-1105 (2010).

Protein expression patterns can be evaluated using any method that provides a quantitative measure and is suitable for evaluation of multiple markers extracted from samples. Exemplary methods include: ELISA sandwich assays, mass spectrometric detection, calorimetric assays, binding to a protein array (e.g., antibody array), or fluorescent activated cell sorting (FACS). Approaches can use labeled affinity reagents (e.g., antibodies, small molecules, etc.) that recognize epitopes of one or more protein products in an ELISA, antibody array, or FACS screen.

Typically, the term high-throughput refers to a format that performs at least about 100 assays, or at least about 500 assays, or at least about 1000 assays, or at least about 5000 assays, or at least about 10,000 assays, or more per day. When enumerating assays, either the number of samples or the number of protein markers assayed can be considered. Generally high-throughput expression analysis methods involve a logical or physical array of either the subject samples, or the protein markers, or both. Appropriate array formats include both liquid and solid phase arrays. For example, assays employing liquid phase arrays, e.g., for hybridization of nucleic acids, binding of antibodies or other receptors to ligand, etc., can be performed in multiwell or microtiter plates. Microtiter plates with 96, 384, or 1536 wells are widely available, and even higher numbers of wells, e.g., 3456 and 9600 can be used. In general, the choice of microtiter plates is determined by the methods and equipment, e.g., robotic handling and loading systems, used for sample preparation and analysis.

Alternatively, a variety of solid phase arrays can also be employed to determine expression patterns. Exemplary formats include membrane or filter arrays (e.g., nitrocellulose, nylon), pin arrays, and bead arrays (e.g., in a liquid "slurry"). Essentially any solid support capable of withstanding the reagents and conditions necessary for performing the particular expression assay can be utilized. For example, functionalized glass, silicon, silicon dioxide, modified silicon, any of a variety of polymers, such as (poly)tetrafluoroethylene, (poly)vinylidenedifluoride, polystyrene, polycarbonate, or combinations thereof can all serve as the substrate for a solid phase array.

It is disclosed that arrays can include "chips" composed, e.g., of one of the above-specified materials. Polynucleotide probes, e.g., RNA or DNA, such as cDNA, synthetic oligonucleotides, and the like, or binding proteins such as antibodies or antigen-binding fragments or derivatives thereof, that specifically interact with expression products of individual components of the candidate library are affixed to the chip in a logically ordered manner, i.e., in an array. In addition, any molecule with a specific affinity for either the sense or anti-sense sequence of the marker nucleotide sequence (depending on the design of the sample labeling), can be fixed to the array surface without loss of specific affinity for the marker and can be obtained and produced for array production, for example, proteins that specifically recognize the specific nucleic acid sequence of the marker, ribozymes, peptide nucleic acids (PNA), or other chemicals or molecules with specific affinity.

Detailed discussion of methods for linking nucleic acids and proteins to a chip substrate, are found in, e.g., U.S. Pat. Nos.5,143,854; 6,087,112; 5,215,882; 5,707,807; 5,807,522; 5,958,342; 5,994,076; 6,004,755; 6, 048,695; 6,060,240; 6,090,556; and 6,040,138.

Microarray expression may be detected by scanning the microarray with a variety of laser or CCD-based scanners, and extracting features with software packages, for example, Imagene (Biodiscovery, Hawthorne, CA), Feature Extraction Software (Agilent), Scanalyze (Eisen, M. 1999. SCANALYZE User Manual; Stanford Univ., Stanford, Calif. Ver 2.32.), or GenePix (Axon Instruments).

It is disclosed that quantitative data obtained about the markers of interest and other dataset components can be subjected to an analytic process with chosen parameters. The parameters of the analytic process may be those disclosed herein or those derived using the guidelines described herein. The analytic process used to generate a result may be any type of process capable of providing a result useful for classifying a sample, for example, comparison of the obtained dataset with a reference dataset, a linear algorithm, a quadratic algorithm, a decision tree algorithm, or a voting algorithm. The analytic process may set a threshold for determining the probability that a sample belongs to a given class. The probability preferably is at least 50%, or at least 60%, or at least 70%, or at least 80%, or at least 90% or higher.

The following examples further illustrate the present disclosure.

## EXAMPLES

### Example 1. Microarray study.

The research described in this Example was approved by the Health Science Board of Hungary and the Human Investigation Committee of Wayne State University. After obtaining informed consent, placental tissue samples were collected from Caucasian women at the First Department of Obstetrics and Gynecology, Semmelweis University. Specimens and data were stored anonymously. Pregnancies were dated to be between 8-12 weeks of gestation according to ultrasound scans. Patients with multiple pregnancies (twins, triplets, etc.) or fetuses having congenital or chromosomal abnormalities were excluded. Women were enrolled in the following homogenous groups: (1) preterm severe preeclampsia, with or without HELLP syndrome (n=12) and (2) preterm controls (n=5) (Table 1). Preeclampsia was defined according to the criteria set by the American College of Obstetricians and Gynecologists (Blood pressure: 140 mm Hg or higher systolic or 90 mm Hg or higher diastolic after 20 weeks of gestation in a woman with previously normal blood pressure; proteinuria: 0.3 g or more of protein in a 24-hour urine collection (usually corresponds with 1+ or greater on a urine dipstick test). Severe preeclampsia was defined according to Sibai et al., [Sibai, B et al. Pre-eclampsia. Lancet 2005;365:785-99]. Preterm controls had no medical complications, clinical or histological signs of chorioamnionitis, and delivered neonates with a birth weight appropriate-for-gestational age (AGA). C-section was performed in all preeclampsia cases due to severe symptoms, as well as in all controls due to previous C-section or malpresentation before 37 weeks of gestation.

Table 1

| Groups | Preterm control (n=5) | Preterm preeclampsia with / without HELLP syndrome (n=12) |
|---|---|---|
| Maternal age (y)[b] | 31.6 (31.5-34.3) | 30.3 (26.1-35) |
| Primiparity[a] | 40 | 66.7 |
| Gestational age (week)[b] | 31.0 (30.9-34.0) | 31.2 (29.3-33.2) |
| Race[a] | | |

(continued)

| Groups | Preterm control (n=5) | Preterm preeclampsia with / without HELLP syndrome (n=12) |
|---|---|---|
| Caucasian | 100 | 100 |
| African American | 0 | 0 |
| Other | 0 | 0 |
| Systolic BP (mmHg)[b] | 120 (120-120) | 163 (160-170)[c] |
| Diastolic BP (mmHg)[b] | 80 (70-80) | 100 (100-101)[c] |
| Proteinuria[a] | 0 | 100 |
| Birth weight (g)[b] | 1990 (1640-2210) | 1065 (990-1420) |
| Cesarean delivery[a] | 100 | 100 |
| [a] Percentage [b] Median (IQR) [c] p<0.01 | | |

**RNA isolation and microarray experiments**

Placentas (n=17) were obtained immediately after delivery. Tissue specimens were excised from central cotyledons close to the umbilical cord in order to reduce the possible bias due to regional differences in gene expression, dissected from the choriodecidua on dry ice and stored at -80°C. Tissues were homogenized using a ThermoSavant FastPrep FP120 Homogenizer (Thermo Scientific, Wilmington, DE, USA) with Lysing MatrixD (MP Biomedicals, Illkirch, France). Total RNA was isolated using RNeasy Fibrous Tissue Mini Kit (Qiagen GmbH, Hilden, Germany), quantified with NanoDrop1000 (Thermo Scientific) and assessed by Agilent 2100 Bioanalyzer (Matriks AS, Oslo, Norway). Total RNAs (controls, n=5; preeclampsia, n=12) were labeled, and Cy3-RNAs were fragmented and hybridized to the Whole Human Genome Oligo Microarray G4112A on an Agilent scanner, (Agilent Technologies, Santa Clara, CA, USA), and processed with Agilent Feature Extraction software v9.5 according to the manufacturer's guidelines.

**Data analysis**

Demographics data were compared by the Fisher's exact test and Mann-Whitney test using SPSS version 12.0 (SPSS Inc., Chicago, IL, USA). Microarray data analysis was performed using the R statistical language and environment (website r-project.org). Microarray expression intensities were background-corrected using the "minimum" method in the "backgroundCorrect" function of the "limma" package. After $\log_2$ transformation, data were quantile-normalized. From the 41,093 probesets on the array, 93 were removed before differential expression analysis because of lacking annotation in the array definition file (Agilent Technologies). Subsequently, an expression filter was applied to retain probesets with intensity greater than $\log_2$ in at least two samples, yielding a final matrix of 30,027 probesets (15,939 unique genes). Differential gene expression was assessed using a moderated t-test. P-values were adjusted using the false discovery rate (FDR) method. Target gene Entrez IDs for the probesets were determined using the R package "hgu4112a.db". For probesets without annotation in the package, Entrez IDs were taken from the array definition file (Agilent Technologies). Probesets remaining un-annotated (without Entrez ID and/or gene symbol) were removed from further analysis. Probesets were defined as differentially expressed (n=1409) in this example if they had a FDR of $\leq 0.2$ and a fold-change of $\geq 1.5$. As used herein, "differential expression", "significantly differentially expressed", and similar terms generally mean that expression of a gene is significantly different based on a statistical power analysis, the results of which can be validated by qPCR at a 95% confidence interval.

The human U133A/GNF1 H microarray data on 79 human tissues, cells and cell lines from Symatlas microarray database [Su, AI et al. A gene atlas of the mouse and human protein-encoding transcriptomes. PNAS 2004;101:6062-67] was downloaded to search for human genes with predominant placental expression. A probeset was defined as having predominant placental expression, if its placental expression was 1) $\geq 1,000$ fluorescence units; 2) six times higher than the 75th quantile of values in 78 other tissue and cell sources; and 3) two times higher than its expression in the tissue with the second highest expression. The resulting 215 probesets corresponded to 153 unique genes. An additional eleven genes not present on the microarray platform (Affymetrix, Santa Clara, CA, USA) used by Symatlas were added to this list based on their potential relevance. Out of 164 predominantly placental expressed genes, 157 were present on our Agilent array. These genes were tested for enrichment in differentially expressed genes compared to all genes on the array (1,409 out of 15,939) using Fisher's exact tests.

Chromosomal locations for all genes tested on the Agilent array were obtained from the R package "org.Hs.eg.db". Out of the 15,939 unique and 1,409 differentially expressed genes on the array, 15,935 and 1,408 could be assigned to chromosomes, respectively. Mapping the microarray probe sets on the Affymetrix human U133A/GNF1 H chips to ENTREZ identifiers was performed using the Bioconductor hgu133a.db and hgfocus.db packages. Chromosomal locations of the resulting list of genes were obtained from the package org.Hs.eg.db and from NCBI for the eleven additional genes. Enrichment analyses for chromosomes among PPE genes, differentially expressed genes, and differentially expressed genes encoding for transcriptional regulators were tested by Fisher's exact test. Chromosomal locations of PPE genes and differentially expressed genes (transcription regulators and non-transcription regulators) were visualized by Circos (Figure 1).

Weighted gene co-expression network analysis (WGCNA) was applied on the 1,409 differentially expressed genes across 17 samples to identify distinct regulation modules and prioritize candidate genes for qPCR verification. Gene pair-wise similarity (absolute Pearson correlation) matrix was first computed, then soft-thresholded by raising to the power of 10 (chosen based on the scalefree topology criterion) to obtain an adjacency matrix. The topology overlap matrix (TOM) was then derived from the adjacency matrix. The topology overlap measures the node interconnectedness within a network and was generalized to a weighted co-expression network. This measure defines similarity between two genes based on both correlations within themselves and outside with other genes. Gene distance matrix was defined as 1-TOM, and used for average linkage hierarchical clustering. A hybrid dynamic tree-cutting method was applied to obtain modules (tree clusters). Gene modules identified with this approach were further tested for enrichment in PPE genes using a Fisher's exact test. Transcription regulatory genes that were expressed at high levels (average $\log_2$ intensity >9) and co-expressed (absolute Pearson coefficient >0.8) with the most genes among PPE genes were treated as candidates for hub-genes in the module.

## Example 2. Validation study.

### Study groups, clinical definitions and sample collection

The research described in this Example was approved by the Institutional Review Boards of the *Eunice Kennedy Shriver* National Institute of Child Health and Human Development (NICHD), National Institutes of Health (NIH), Department of Health and Human Services (DHHS), and Wayne State University. After informed consent was obtained, placentas (n=100) were retrieved from the bank of biological specimen of the Perinatology Research Branch (NICHD, NIH, DHHS). Pregnancies were dated to be between 8-12 weeks according to ultrasound scans. Patients with multiple pregnancies (twins, triplets, etc.) or fetuses having congenital or chromosomal abnormalities were excluded. Specimens and data were stored anonymously.

For qRT-PCR, tissue microarray, mRNA in situ hybridization, and laser capture microdissection, placentas were used from women selected from a large cohort into the following, homogenous patient groups: (1) preterm severe preeclampsia (PE; ≤36 weeks; n=20); (2) preterm severe preeclampsia associated with small-for-gestational age (SGA) (PE-SGA; ≤36 weeks; n=20); (3) preterm controls (PTC; ≤36 weeks; n=20); (4) term severe preeclampsia (TPE; ≥37 weeks; n=10); (5) term severe preeclampsia associated with SGA (TPESGA; ≥37 weeks; n=10); and (6) term controls (TC; ≥37 weeks; n=20). Women in these groups were predominantly of African American origin (Table 2). Term controls, consisting of normal pregnant women with (n=10) or without (n=10) labor, and preterm controls with preterm labor and delivery (n=20) had no medical complications or clinical or histological signs of chorioamnionitis, and delivered AGA neonates. Labor was defined by the presence of regular uterine contractions at a frequency of at least two contractions every 10 minutes with cervical changes resulting in delivery. Preeclampsia was defined according to the criteria set by the American College of Obstetricians and Gynecologists. Severe preeclampsia was defined according to Sibai et al., see above. SGA was defined as neonatal birth-weight below the 10th percentile for gestational age. C-section was performed in all preeclampsia cases due to severe symptoms and in controls due to previous C-section or malpresentation.

Table 2

| Groups | Preterm control (n=20) | Preterm preeclampsia (n=20) | Preterm preeclampsia with SGA (n=20) | Term control (n=20) | Term preeclampsia (n=10) | Term preeclampsia with SGA (n=10) |
|---|---|---|---|---|---|---|
| Maternal age (y)[b] | 22 (20-28.5) | 23.5 (21-27) | 22.5 (19.5-30) | 22 (21-32) | 19 (19-35) | 26.5 (19-31) |
| Primiparity[a] | 20 | 40 | 25 | 15 | 40 | 10 |

(continued)

| Groups | Preterm control (n=20) | Preterm preeclampsia (n=20) | Preterm preeclampsia with SGA (n=20) | Term control (n=20) | Term preeclampsia (n=10) | Term preeclampsia with SGA (n=10) |
|---|---|---|---|---|---|---|
| Gestational age (week)[b] | 32.3 (28.2-34.9) | 31.4 (29.6-33.6) | 31.8 (29.7-34.4) | 38.6 (38-39.1) | 39.1 (38.6-39.6) | 38.4 (37.3-38.9) |
| Groups | Preterm control (n=20) | Preterm preeclampsia (n=20) | Preterm preeclampsia with SG A (n=20) | Term control (n=20) | Term preeclampsia (n=10) | Term preeclampsia. with SG A (n=10) |
| Race[a] | | | | | | |
| Caucasian | 5 | 10 | 10 | 15 | 0 | 0 |
| African American | 95 | 90 | 90 | 80 | 100 | 100 |
| Other | 0 | 0 | 0 | 5 | 0 | |
| Systolic BP (mmHg)[b] | 116 (110-125) | 177 (166-187)[c] | 171 (164-189)[c] | 121 (111-134) | 173 (165-178)[c] | 169 (164-190)[c] |
| Diastolic (mmHg)[b] | 65 (59-71) | 105 (103-111)[c] | 108 (94-118)[c] | 70 64-73) | 106 (102-110)[c] | 102 (97-104)[c] |
| Proteinuria[b] | 0 | 3 (2-3)[c] | 3 (3-3)[c] | 0 | 3 (1-3)[c] | 3 (1-3)[c] |
| Birth weight (g)[b] | 1635 (1075-2715) | 1488 (1050-1908) | 1173 (908-1650) | 3215 (3110-3335) | 3123 (2990-3200) | 2405 (2205-2555)[c] |
| weight percentile[b] | 40.5 (31.9-53.4) | 22.7 (18.3-32.9)[d] | 6.7 (1-8.6)[c] | (37.2-63) | 37.1 (28.5-48.8) 48.8) | 1.1 (1-3.5)[c] |
| Cesarean delivery[a] | 45 | 80[d] | 75 | 55 | 40 | 20 |
| [a] Percentage; [b] Median (IQR); [c] p<0.001; [d] p<0.05 | | | | | | |

## Total RNA isolation, cDNA generation and quantitative real-time RT-PCR

Total RNA was isolated from snap-frozen placental villous tissues (n=100) with TRIzol reagent (Invitrogen, Carlsbad, CA, USA) and Qiagen RNeasy kit (Qiagen, Valencia, CA, USA) according to the manufacturers' recommendations. The 28S/18S ratios and the RNA integrity numbers were assessed using an Agilent Bioanalyzer 2100 (Agilent Technologies), RNA concentrations were measured with NanoDrop1000 (Thermo Scientific). Five hundred ng of total RNA was reverse transcribed with High Capacity cDNA Reverse Transcription Kit using random hexamers (Applied Biosystems). TaqMan Assays (Applied Biosystems) were used for high-throughput gene expression profiling on the Biomark™ qRT-PCR system (Fluidigm, San Francisco, CA, USA) according to the manufacturers' instructions.

## Tissue microarray (TMA) construction, immunostaining and immunoscoring

TMAs were constructed from FFPE villous tissue blocks (n=100). Briefly, three 20x35mm recipient blocks were made of Paraplast X-Tra tissue embedding media (Fisher Scientific, Pittsburgh, PA, USA). One mm diameter cores from tissue blocks were transferred in triplicate into recipient paraffin blocks using an automated tissue arrayer (Beecher Instruments, Inc., Silver Spring, MD, USA). Five $\mu$m sections cut from TMAs were placed on silanized slides and stained with antibodies and reagents manually, or either on a Ventana Discovery autostainer (Ventana Medical Systems, Inc, Tucson, AZ, USA) or a Leica BOND-MAX™ autostainer (Leica Microsystems, Wetzlar, Germany). Images were captured with an Olympus BX41 microscope (Olympus America Inc., Center Valley, PA, USA). immunostainings were semiquantitatively scored by two examiners blinded to the clinical information with a modified immunoreactive score. Immunostaining intensity was graded as follows: 0=negative, 1=weak, 2=intermediate, and 3=strong. All villi in a random field of each of three cores were evaluated by both examiners, and scores within each core were averaged to represent target protein quantity of that core. Thus, each placenta had three scores corresponding to three cores examined, and group comparisons using these scores were conducted in a same way as for qRT-PCR data.

**Histopathologic evaluation of the placenta**

Placental tissue samples (n=100) were taken by systematic random sampling, fixed in 10% neutral-buffered formalin, and embedded in paraffin. Five $\mu$m sections were cut from the villous tissue blocks, stained with hematoxylin and eosin, and examined using bright-field light microscopy by two anatomic pathologists blinded to the clinical information. Histopathologic changes were defined according to published criteria. "Maternal underperfusion" and "fetal vascular thrombo-occlusive disease" scores were calculated by summing the number of different pathologic lesions consistent with these lesion categories present in a given placenta.

**Statistical analysis and evaluation of qRT-PCR data**

Demographics data were compared by the Fisher's exact test and Mann-Whitney test using SPSS version 12.0 (SPSS). qPCR data were analyzed using the $\Delta\Delta$Ct method in the R statistical environment (website r-project.org). Data was first normalized to the reference gene *(RPLPO)* and batch effect was adjusted through calibrator samples. $Log_2$mRNA relative concentrations were obtained for each sample as $-\Delta Ct_{(gene)} = Ct_{(RPLPO)} - Ct_{(gene)}$. The surrogate gene expression values ($-\Delta Ct_{gene}$) were used to perform a hierarchical clustering with 1-Pearson correlation distance and average linkage. Between group comparisons (in which groups were predefined based on the clinical characteristics of the patients) were performed by fitting a linear model on $-\Delta Ct$ values, using as covariates the group variable indicator while allowing for an interaction between the group variable and the maturity status of the fetus (term vs. preterm).

Besides these group comparisons, the analysis was extended to include all 100 patients in the validation phase, to test for the association between gene expression and blood pressure as well as birth weight while adjusting for gestational age. All variables in the latter analysis were treated as continuous. P-values of <0.05 were considered significant.

A neural networks based approach was used to determine the best combination of 2-8 genes that would best predict blood pressure and birth weight at the same time based on qRT-PCR data. Samples (n=100) were randomly split into 10 equal and balanced (with respect to the presence of preeclampsia) cross-validation folds. At each fold, 90% of the samples were used to rank the genes in an univariate fashion for predicting blood pressure and birth weight separately, and the best 15 genes for each of the two outcomes were retained using simple linear model, adjusting for gender and maturity (term/preterm). Then all gene-combinations were used as inputs in a neural network model that was trained to predict both blood pressure and birth weight using the training data. The remaining 10% of the samples were used to determine the Average Absolute Relative prediction Error (AARE) of the neural network for each gene-combination. The cross-validation procedure was repeated 10 times, splitting therefore the data into 10 different 10-fold partitions, for a total of 100 training and 100 test sets of samples. The number of times that a given gene-combination was found in the top 5% of combinations (the smallest AARE) was recorded and used to rank the combinations of genes for their ability to predict both blood pressure and birth weight percentile. A linear discriminant analysis (LDA) model was used to provide a realistic measure of the sensitivity and specificity of a model predicting the disease status (preeclampsia vs controls). LDA was performed starting with six genes (*FLT1, HSD17B1, LEP, LGALS14, PAPPA2,* and *PLAC1*) chosen from the results of the neural network analysis as being top 2 best predictors and / or highly placenta specific genes, and then was also repeated with a restricted set of four genes (*HSD17B1, LGALS14, PLAC1,* and *PAPPA2)*. The 100 samples were split repeatedly at random in two parts: a training part (80% of the samples) was used to fit a LDA model, and a test part (20% of the samples) was used to compute the sensitivity and specificity of the fitted model. The estimates for sensitivity and specificity were averaged over 100 such trials to give a robust estimate. At each trial, the genes considered were ranked using a t-test and the optimal number of them to be included in the LDA model is determined using the performance of LDA model via an internal 3-fold cross-validation process. The procedure is described in more detail elsewhere (Tarca, A.L., Than, N.G., & Romero, R. Methodological Approach from the Best Overall Team in the IMPROVER Diagnostic Signature Challenge. Systems Biomedicine submitted, (2013).

The model had two sets of parameters. The mean expressions values ($-\Delta Ct$ values) of the four genes in the two groups were (m):

|  | HSD 17B 1 | LGALS14 | PLAC 1 | PAPPA2 |
|---|---|---|---|---|
| **m**$_{Ct1}$ | 0.091715 | -1.77314 | -1.81852 | -0.65007 |
| **m**$_{PE}$ | -1.05794 | -2.8269 | -2.69142 | 1.092378 |

and the variance-covariance matrix (E) was:

|  | HSD17B1 | LGALS14 | PLAC1 | PAPPA2 |
|---|---|---|---|---|
| HSD17B1 | 1.677801 | 1.583045 | 1.354269 | 0.26431 |
| LGALS14 | 1.583045 | 2.082332 | 1.533739 | 0.551178 |
| PLAC1 | 1.354269 | 1.533739 | 1.654183 | 0.331429 |
| PAPPA2 | 0.26431 | 0.551178 | 0.331429 | 1.904354 |

For any new individual we assumed that the expression profile x was available e.g. x=

| HSD17B1 | LGALS14 | PLAC1 | PAPPA2 |
|---|---|---|---|
| -1.29723 | -2.68723 | -2.9415 | 2.099069 |

The posterior probability for each patient class (preeclampsia vs. controls) was computed from the new profile values and model parameters using the multivariate normal formula:

$$p(\mathbf{x}\,|\,Ctl) = \frac{1}{(2\pi)^{N/2}\,|\Sigma|^{1/2}}\exp\left(-\frac{1}{2}(\mathbf{x}-\mathbf{\mu}_{Ctl})^{T}\Sigma^{-1}(\mathbf{x}-\mathbf{\mu}_{Ctl})\right) = 0.00149$$

$$p(\mathbf{x}\,|\,PE) = \frac{1}{(2\pi)^{N/2}\,|\Sigma|^{1/2}}\exp\left(-\frac{1}{2}(\mathbf{x}-\mathbf{\mu}_{PE})^{T}\Sigma^{-1}(\mathbf{x}-\mathbf{\mu}_{PE})\right) = 0.0196$$

When p(x|PE)> p(x|Ctl), the sample was classified as preeclampsia. When p(x|Ctl)>p(x|PE), the sample was classified as control.

The statistical R package was used to compute these probabilities using the following syntax, assuming that these parameters are loaded into R first, and the *mvtnorm* library is also loaded:

| > X |  |  |  |  |
|---|---|---|---|---|
|  | HSD17B1 | LGALS141 | PLAC1 | PAPPA2 |
| 10796 | -1.297228 | -2.687228 | -2.941502 | 2.099069 |
| m |  |  |  |  |
|  | HSD 17B 1 | LGALS14 | PLAC1 | PAPPA2 |
| Control | 0.091715 | -1.773139 | -1.818516 | -0.650065 |
| PE | -1.057936 | -2.826896 | -2.691423 | 1.0923776 |
| > sigma |  |  |  |  |
|  | HSD17B1 | LGALS14 | PLAC1 | PAPPA2 |
| HSD17B1 | 1.6778008 | 1.5830448 | 1.3542685 | 0.2643097 |
| LGALS14 | 1.5830448 | 2.0823321 | 1.5337394 | 0.5511775 |
| PLAC 1 | 1.3542685 | 1.5337394 | 1.6541829 | 0.3314286 |
| PAPPA2 | 0.2643097 | 0.5511775 | 0.3314286 | 1.9043535 |
| > pCtl=dmvnorm(nx, m[1,], sigma,log=FALSE) |  |  |  |  |
| > pPE=dmvnorm(nx, m[2,], sigma,log=FALSE) |  |  |  |  |
| > pCtl |  |  |  |  |
|  | 10796 |  |  |  |

(continued)

|  | HSD17B1 | LGALS14 | PLAC1 | PAPPA2 |
|---|---|---|---|---|
| 0.001489049 |  |  |  |  |
| > pPE |  |  |  |  |
|  | 10796 |  |  |  |
| 0.01960817 |  |  |  |  |

## Example 3. Maternal serum proteomics

### Study groups, clinical definitions and sample collection

All women were enrolled in a prospective, longitudinal, multicenter study in prenatal community clinics of the Maccabi Healthcare Services, Israel between August 2002 and March 2003. Pregnancies were dated according to the last menstrual period and verified by first trimester ultrasound. Patients with multiple pregnancies (twins, triplets, etc.) or fetuses having congenital or chromosomal abnormalities were excluded. The collection and investigation of human clinical samples were approved by the Maccabi Institutional Review Board, experimental procedures and data analyses were approved by the Health Science Board of Hungary and the Human Investigation Committee of Wayne State University. Informed consent was obtained from women prior to sample collection. Specimens and data were stored anonymously.

Preeclampsia was defined as hypertension that developed after 20 weeks (systolic or diastolic blood pressure $\geq$140 or $\geq$90 mmHg, respectively, measured at two different time points, 4h to 1 week apart) coupled with proteinuria ($\geq$300mg in a 24h urine collection or $\geq$2+ on a dipstick) according to the International Society for the Study of Hypertension in Pregnancy. Preeclampsia was defined severe, if 1) severe hypertension (systolic or diastolic blood pressure $\geq$160 or $\geq$110 mmHg) was coupled with proteinuria; 2) if hypertension was coupled with severe proteinuria ($\geq$5g/24h or $\geq$3 on a dipstick), or 3) if maternal multi-organ involvement was present, such as pulmonary edema, oliguria, abnormal liver function, epigastric or right upper-quadrant pain, thrombocytopenia, or severe central nervous symptoms including seizures. Small-for gestational age was defined as neonatal birth weight below the 10th percentile for gestational age. Healthy controls had no medical or obstetric complications and delivered a neonate with a birth-weight appropriate for gestational age.

Peripheral blood samples were obtained by venipuncture in the first trimester from women who subsequently developed preterm severe preeclampsia (<36 weeks; n=5), term severe preeclampsia ($\geq$37 weeks; n=5), as well as healthy controls ($\geq$37 weeks; n=10) matched for gestational age at blood draw (Table 3). Samples were kept for 1-2h at room temperature (RT) and then centrifuged at 10,000g for 10min. Sera were collected, stored at 2-8°C for up to 48h until transferred to the Maccabi Central Laboratory, and then stored in aliquots at -20°C until shipped on dry ice to Hungary.

Table 3.

| Groups | Controls for PE with SGA | Preeclampsia with SGA | Controls for term PE | Term preeclampsia |
|---|---|---|---|---|
| Number of cases | 5 | 5 | 5 | 5 |
| Gestational age at blood draw (week) | 10 (9-11) | 8 (8-9) | 8 (8-9) | 9 (8-10) |
| Gestational age at delivery (week) | 39.7 (38.6-40.0) | 34.9 (29.3-35.3) | 38.7 (38.6-41.0) | 38.1 (38.0-38.1) |
| Systolic BP (mmHg) | 105 (104-110) | 160 (150165) | 110 (110118) | 150 (140160) |
| Diastolic BP (mmHg) | 60 (60-70) | 100 (100-100) | 67 (63-68) | 100 (90-100) |
| Proteinuria | 0 | 4 (3-4) | - | 3 (3-4) |
| Birth weight (gram) | 2955 (2900-3100) | 1720 (975-1800) | 2955 (2900-3100) | 3200 (3150-3210) |
| Median (IQR) | | | | |

## I. Discovery phase

### Sample preparations, immunodepletion of high-abundance serum proteins

Sera were immunodepleted at Biosystems International Ltd. (Debrecen, Hungary) for 14 highly abundant serum proteins on an Agilent 1100 HPLC system using Multiple Affinity Removal LC Column -Human 14 (Agilent Technologies, Santa Clara, CA, USA) according to the manufacturer's protocol. To improve the resolution of 2D gels, immunodepleted serum samples were liophylized, and then delipidated and salt depleted at Proteome Services, Ltd. (Budapest, Hungary). Briefly, one volume of all samples was mixed with four volumes of methanol and was thoroughly vortexed. Subsequently, one volume of chloroform was added to these mixtures, which were vortexed again followed by the incorporation of three volumes of water (HPLC grade). After centrifugation at 14,000rpm for 5min at 4°C, the upper phases were discarded. Three volumes of methanol were then added and the resultant mixtures were vortexed and centrifuged again. The supernatants were discarded and the pellets containing the precipitated plasma proteins were air-dried for 10min. The delipidated and salt-depleted plasma protein samples were dissolved in lysis buffer (7M urea; 2M thiourea; 20mM Tris; 5mM magnesium acetate, 4% CHAPS) and their pH was adjusted to 8.0.

### Fluorescent labeling and two-dimensional differential in-gel electrophoresis (2D-DIGE)

Protein concentrations of the immunodepleted, desalted and delipidated serum samples were between 2-4$\mu$g/$\mu$l as determined with PlusOne Quant Kit (GE Healthcare, Pittsburgh, PA, USA). Samples were equalized for protein content, and then 5$\mu$g of each protein sample was labeled with CyDye DIGE Fluor Labeling kit for Scarce Samples (saturation dye) (GE Healthcare) at a concentration of 4nmol/5$\mu$g protein according to the manufacturer's instructions. Individual samples from cases (n=10) and controls (n=10) were labeled with Cy5. An internal standard reference sample was pooled from equal amounts (25$\mu$g) of all individual samples in this experimental set and was labeled with Cy3. Then, 5$\mu$g of each Cy5-labeled individual sample was merged with 5$\mu$g of the Cy3-labeled reference sample, and these 20 mixtures were run in 2x10 gels simultaneously. Briefly, labeled proteins were dissolved in IEF buffer containing 0.5% ampholytes, 0.5% DTT, 8M urea, 30% glycerin, 2% CHAPS and were rehydrated passively onto 24cm IPG 20 strips (pH3-10, GE Healthcare) for at least 14h at RT. After rehydration, the IPG strips were subjected to first dimension IEF for 24h to attain a total of 80kVh. Focused proteins were reduced by equilibrating with a buffer containing 1% mercaptoethanol for 20min. After reduction, IPG strips were loaded onto 10% polyacrylamide gels (24x20cm) and SDS-PAGE was conducted at 10W/gel in the second dimension. Then, gels were scanned in a Typhoon TRIO+ scanner (GE Healthcare) using appropriate lasers and filters with the PMT biased at 600V. Images in different channels were overlaid using selected colors and the differences were visualized using Image Quant software (GE Healthcare). Differential protein analysis was performed using the Differential In-gel Analysis (DIA) and Biological Variance (BVA) modules of the DeCyder 6.0 software package (GE Healthcare).

### Identification of differentially expressed protein spots

The internal standard reference sample representative of every protein present in all experiments was loaded equally in all gels, and thus, provided an average image for the normalization of individual samples. The determination of the relative abundance of the fluorescent signal between internal standards across all gels provided standardization between the gels, removing experimental variations and reducing gel-to-gel variations. According to the standard proteomic protocol, the threshold for differential expression was set at 1.05-fold minimum fold-change. A p-value was determined for each protein spot using the Student's t-test by the BVA module of the DeCyder software (GE Healthcare). A p-value of <0.05 was considered statistically significant.

## II. Preparative phase

### Sample preparation, fluorescent labeling, 2D-DIGE

The density of spots in the case of Colloidal Coomassie Blue labeling depends only the concentration of protein in the sample, however the density of spots in the case of saturation dyes labeling depends on the number of cysteines of the labeled proteins too, because the saturation dyes labeling method labels all available cysteines on each protein. This results in the same pattern with different density among samples on the analytical and the preparative gels rendering identification more difficult. To eliminate this problem for the exact identification of proteins in spots of interest, the preparative 2D electrophoresis was performed using CyDye saturation fluorescent labeling and Colloidal Coomassie Blue labeling in the same gel. A total of 800$\mu$g of proteins per each of the two gels ran. Briefly, the 10-10 immunodepleted serum samples in the "preterm" and "term" comparisons were pooled together and the salt-depletion step was repeated

three-times. Five μg protein from each of these two pooled samples was labeled with Cy3, merged with 800μg of unlabeled proteins from the same sample and resolved in the dry-strip. After separation of the first dimension, focused proteins were first reduced by equilibrating with a buffer containing 1% mercaptoethanol for 20min, and then alkylated with a buffer containing 2.5% iodoacetamide for 20min. Following electrophoresis, gels were scanned in a Typhoon TRIO+ scanner as described above, the differentially expressed spots were matched among the "master" analytical and the fluorescent preparative gel image using Biological Variance (BVA) modules of the DeCyder 6.0 software package (GE Healthcare). The resolved protein spots were visualized by the Colloidal Coomassie Blue G-250 staining protocol. Differentially expressed individual spots were excised from the gels to compare the images.

## III. Identification phase

### In-gel digestion

The excised protein spots were analyzed at the Proteomics Research Group of the Biological Research Center of the Hungarian Academy of Sciences (Szeged, Hungary); the detailed protocol is entitled "In-Gel Digest Procedure" described in the website "msfacility.ucsf.edu/ingel.html" and reproduced below: Briefly, salts, SDS and Coomassie brilliant blue were washed out, disulfide bridges were reduced with dithiothreitol, and then free sulfhydryls were alkylated with iodoacetamide. Digestion with side-chain protected porcine trypsin (Promega) proceeded at 37°C for 4h, and the resulting peptides were extracted.

### In-Gel Digest Procedure

1. 1. Wearing gloves and sleeve protectors, wipe down ALL surfaces in the hood with methanol/water moistened lint-free cloth, including the outside of all your tubes (make sure to not wipe off the labeling!), the outside and inside of the Speed Vac and centrifuge, tube racks, bottles etc. Wipe razor blades with methanol-soaked lint-free cloth.
2. 2. Prepare the following solutions:

   25 mM $NH_4HCO_3$ (100 mg/50 ml)
   25 mM $NH_4HCO_3$ in 50% ACN
   50% ACN/5% formic acid (may substitute TFA or acetic acid)
   12.5 ng/μL trypsin in 25mM $NH_4HCO_3$ (freshly diluted)

3. 3. Dice each gel slice into small pieces (1 mm2) and place into 0.65 mL siliconized tubes (PGC Scientific).
4. 4. Add ~100μL (or enough to cover) of 25mM $NH_4HCO_3$/50% ACN and vortex for 10 min.
5. 5. Using gel loading pipet tip, extract the supernatant and discard.
6. 6. Repeat steps 3 and 4 once or twice.
7. 7. Speed Vac the gel pieces to complete dryness (~ 20 min). *Forlow-level proteins (<1 pmol), especially those separated by 1-D SDS-PAGE, reduction and alkylation is recommended. These procedures are performed after step* 6.

   1. a. Prepare fresh solutions:

      10 mM DTT in 25 mM $NH_4HCO_3$ (1.5 mg/mL)
      55 mM iodoacetamide in 25 mM $NH_4HCO_3$ (10 mg/mL)

   2. b. Add 25 μL (or enough to cover) 10 mM DTT in 25 mM $NH_4HCO_3$ to dried gels. Vortex and spin briefly. Allow reaction to proceed at 56°C for 1 hr.
   3. c. Remove supernatant, add 25 μl 55 mM iodoacetamide to the gel pieces. Vortex and spin briefly. Allow reaction to proceed in the dark for 45 min. at room temperature.
   4. d. Remove supernatant (discard). Wash gels with ~100 μl $NH_4HCO_3$, vortex 10 min, spin.
   5. e. Remove supernatant (discard). Dehydrate gels with ~100μL (or enough to cover) of 25 mM $NH_4HCO_3$ in 50% ACN, vortex 5 min, spin. Repeat one time.
   6. f. Speed Vac the gel pieces to complete dryness (~20 min). Proceed with trypsin digest.

8. 8. Add trypsin solution to just barely cover the gel pieces. Estimate the gel volume and add about 3x volume of trypsin solution. This volume will vary from sample to sample, but on average ~5-25 μL is sufficient.

9. 9. Rehydrate the gel pieces on ice or at 4°C for 10 min. Spin. Add 25mM $NH_4HCO_3$ as needed to cover the gel pieces.
10. 10. Spin briefly and incubate at 37°C for 4 hours - overnight.

*Extraction of Peptides*

1. 1. Transfer the digest solution (aqueous extraction) into a clean 0.65 mL siliconized tube.
2. 2. To the gel pieces, add 30 $\mu$L (enough to cover) of 50% ACN/5% formic acid, vortex 20-30min., spin, sonicate 5 min. Repeat.
3. 3. Vortex the extracted digests, spin and Speed Vac to reduce volume to 10 $\mu$L.
4. 4. Either proceed with C18 ZipTip (Millipore) cleanup or analyze with LC-MS. Add 2-5 $\mu$L of 5% formic acid. When analyzing low levels of protein, concentrate the petides by eluting from ZipTips using 3$\mu$L of elution solution, into a clean 0.65 mL siliconized tube.
5. 5. Use 1$\mu$L of the unseparated digests for analysis by MALDI.

Matrices for unseparated digests:

$\alpha$-cyano-4-hydroxycinammic acid in 50% ACN/1% TFA (10 mg/mL).
2,5-dihydroxybenzoic acid (DHB), saturated solution in water.

**References:**

Rosenfeld, et al., Anal. Biochem. (1992) 203(1), 173-179.
Hellman, et al., Anal. Biochem. (1995) 224(1), 451-455.

**LC-MS/MS**

Samples were analyzed on a Waters Acquity nanoUPLC system online coupled to an ion trap tandem mass spectrometer (LCQ Fleet, ThermoScientific) in information-dependent acquisition mode, where MS acquisitions (1 s survey scans) were followed by CID analyses (3s MS/MS scans) on computer-selected multiply charged ions. HPLC conditions included in-line trapping onto a nanoACQUITY UPLC trapping column (Symmetry, C18 5 $\mu$m, 180 $\mu$m x 20 mm) (15 $\mu$l/min with 3% solvent B) followed by a linear gradient of solvent B (10 to 50% in 40min, flow rate: 250nl/min; nanoACQUITY UPLC BEH C18 Column, 1.7$\mu$m, 75$\mu$m x 200mm). Solvent A: 0.1% formic acid in water, solvent B: 0.1% formic acid in acetonitrile. LC-MS/MS analysis was performed in "triple play" mode in the mass range of m/z: 450-1600.

**Database search and data interpretation**

Raw data files were converted into searchable peak list Mascot generic files (*.mgf) with the Mascot Distiller software v2.1.1.0. (Matrix Science, Inc, London, UK). The resulting peak lists were searched against a human subdatabase of the non-redundant protein database of the National Center for Biotechnology Information (NCBInr 2008.07.18., Bethesda, MD, USA; 6,833,826 sequences) in MS/MS ion search mode on an in-house Mascot server v2.2.04 using Mascot Daemon software v2.2.2. (Matrix Science Inc). Monoisotopic masses with peptide mass tolerance of $\pm$50ppm and fragment mass tolerance of $\pm$0.1 Da were submitted. Carbamidomethylation of Cys was set as fixed modification, and acetylation of protein N-termini, methionine oxidation, and pyroglutamic acid formation from peptide N-terminal Gln residues were permitted as variable modifications. Acceptance criteria was set to at least two significant (peptide score>40, p<0.05) individual peptides per protein.

**RESULTS**

**I. Differentially expressed genes in preeclampsia are enriched among predominantly placental expressed genes and on three chromosomes**

Because the pathogenesis of preeclampsia originates from the placenta, new biomarker candidates predominantly expressed in the placenta as well as gene-regulatory networks involved in the placental pathogenesis of preeclampsia with a systems biological approach were sought. Analysis of a microarray dataset revealed 1,409 differentially expressed unique genes in preterm preeclampsia compared to preterm controls. From these differentially expressed genes, 137 were found to encode for proteins with functions in transcription regulation (transcription factors, co-activators, or co-repressors). Analysis of BioGPS microarray data and previous evidence revealed 164 unique genes predominantly expressed in the placenta, from which 157 were present on our microarray platform.

Differentially expressed genes in preeclampsia were highly enriched (OR=3.4, p=6.9×10$^{-9}$) in PPE genes (38 out of 157) when compared to all genes on the array. When investigating chromosomal locations of genes of interest, it was found that differentially expressed genes were enriched in genes located on Chromosomes 6 and 7 (OR=1.54, pFDR=1.6×10$^{-3}$, and OR=1.42, pFDR=0.02, respectively). Interestingly, Chromosome 19 was over-represented in differentially expressed transcription regulatory genes (OR=2.6, pFDR=0.02), and genes with predominant placental expression (OR=2.5, p=1×10$^{-4}$). These enrichments are in accordance with the fact that Chromosome 19 harbors large primate and placenta-specific gene families (e.g. *CGBs, LGALSs, PSGs*) and zinc finger transcription factor gene families. Visualization of gene expression and co-expression data supports a potential regulatory "hub" role for Chromosome 19 in placental gene expression in primates and its dysregulation in preeclampsia.

Figure 1. Genomic map of differentially expressed genes in preeclampsia. Circos visualization shows Chromosomes with solid lines in the inner circle. Curved lines connect the genomic coordinates of genes and transcription regulatory genes that are significantly correlated. Significance was determined by fitting a linear model between the expression level of gene and transcription regulatory gene pairs in all samples while controlling for FDR at 5%. Curves represent positive and negative correlations. The second circle shows the genomic location of PPE genes (black lines: non differentially expressed; grey lines: up- or down-regulated). The third and fourth circles show the locations of differentially expressed transcription regulatory genes and non-regulatory genes, respectively with inward-oriented bars (down-regulated) and outward oriented bars (up-regulated). The height of the bars in the third and fourth circles represents the magnitude of gene expression changes.

Figure 2. Two gene modules in preeclampsia are enriched in PPE genes and are associated with mean arterial blood pressure and birth weight percentile. 2A) Gene modules identified from WGCNA analysis of microarray data. Dysregulated placental gene expression could be characterized by five gene modules within the 1,409 differentially expressed genes in preeclampsia, marked with different shades of grey. The height plotted on the y-axis represents the distance metric (1-TOM) used by WGCNA. Out of 38 PPE genes (black vertical lines), 33 belonged to the grey-scale modules (lighter grey; n=22 and darker grey; n=11). These two modules were also enriched in up-regulated and down-regulated genes marked under the modules with grey or black lines, respectively). 2B) Hierarchical clustering of qRT-PCR data obtained with 100 samples and selected 47 genes. Genes from light and dark grey modules clustered together in the validation sample-set. Importantly, 34 out of 60 samples from women with preeclampsia clustered tightly together. Pearson correlation was used for distance, and average for linkage. Samples (column leafs) were shaded according to patient groups and maturity status. 2C) Association of gene expression with mean arterial blood pressure and birth weight percentile. For each gene, a linear model was fitted (expression-blood pressure + birth weight percentile + gender + maturity status). The significance p-values (-log$_{10}$of) for the two coefficients (blood pressure and birth weight percentile) were plotted for all 47 genes. Genes were shaded according to module membership (except black color for those not differentially expressed on the microarray). Filled circles represent PPE genes, dashed lines the significance threshold at p=0.05. Note that 7 out of 9 genes related to birth weight percentile are from the light grey module, while 10 out of 15 genes related to blood pressure are from the dark grey module.

## II. Differentially expressed genes in preeclampsia cluster into major regulatory modules

In order to identify regulatory modules of genes and transcription regulatory genes, which may drive dysregulated placental gene expression, a WGCNA analysis with the differentially expressed genes on the microarray was conducted. Out of 1,409, 1,403 genes were assigned to four modules containing 506, 442, 381, and 74 genes. Of interest, 33 out of 38 genes with predominant placental expression belonged to the light grey (n=22) and dark grey (n=11) modules. The light grey module was enriched in down-regulated (OR=1.88, p=2.59x10$^{-8}$), while the dark grey module was enriched in up-regulated (OR=6.47, p<2.2×10$^{-16}$) genes, suggesting the presence of distinct dysregulated gene-networks in preterm preeclampsia.

Among up-regulated genes in the dark grey module was FLT1, which has a pathogenic role in preeclampsia by producing increased amounts of soluble Flt-1 and driving blood pressure elevation. Up-regulated genes with predominant placental expression included *CRH, LEP, PAPPA2, SIGLEC6* and novel biomarker candidates. Among down-regulated, PPE genes in the light grey module were regulators of fetal growth (CSH1, *HSD11B2),* metabolism (ESRRG), estrogen synthesis *(HSD1781),* stress hormone metabolism *(HSD11B2)* and immune regulation of placentation *(LGALS14).*

**III. Differentially expressed genes in the dark grey and light grey modules are associated with blood pressure and birth-weight percentile, respectively**

To validate the described results on a large patient population with different ethnic origin and with various subtypes of preeclampsia (preterm and term, with or without SGA), 47 genes for high-throughput expression profiling were selected, if they were: 1) differentially expressed on the microarray, predominantly placental expressed, specifically by the syncytiotrophoblast, and potentially secreted; 2) transcription regulatory genes with high co-expression with PPE genes; and 3) other genes with relevant role in trophoblast differentiation (e.g. *GCM1*), trophoblast-specific gene expression (e.g. *TEAD3*) or pathogenesis of preeclampsia (e.g. *ENG, LGALS13*).

Hierarchical clustering of qRT-PCR data showed that 34 out of 60 placentas from women with preeclampsia clustered together. This was also true for the genes belonging to the light and dark grey modules (Figure 2B). Based on the possible involvement of these modules in distinct pathogenic pathways, revealing their biological relevance in a novel way was attempted. "Phenotype analysis" showed that 7 out of 9 genes related to birth weight percentile were from the light grey module, while 10 out of 15 genes related to blood pressure were from the dark grey module (Figure 2C).

Placental histopathologic data was also assessed. It was found that the expression of genes in the dark grey module was significantly associated with the presence of "fetal vascular thrombo-occlusive disease" *(SIGLEC6, ENG, TPBG)* and "maternal underperfusion" (top associations: *LEP, FLT1, TPBG, ENG),* conditions consistent with placental hypoxia and/or ischaemia. The majority of the light grey module genes (top associations: *CLDN1, HSD17B1, CSH1, PLAC1, LGALS14)was* significantly associated with the presence of "maternal underperfusion".

In addition, using a classical approach, group comparisons between controls and two groups of preeclampsia at term and preterm separately were performed. It was found that qRT-PCR data validated microarray results in 72% (34/47 genes). Tissue microarray immunostainings for four selected proteins validated the microarray data at the protein level for this module.

Figure 3. The expression of dark grey module genes changes in the same direction in preeclampsia subgroups. 3A-B) In each barplot, the left and right panels show significant differences ("*") in preterm and term preeclampsia samples, respectively. Gene expression 3A) and protein immunostainings 3B) show similar patterns in sub-groups of preeclampsia. When the change with preeclampsia in the preterm samples was significantly different than the change with preeclampsia in the term samples, a "+" sign indicates this interaction. Semiquantitative immunoscorings for four proteins 3B) validated gene expression data. 3C) Representative images from the four immunostainings. The same placenta from a preterm control (left, 29 weeks) and from a patient with preterm preeclampsia with SGA (right, 31 weeks) is shown for the four immunostainings (40x magnifications).

In the light grey module, gene expression changes were also more severe in the preterm groups of preeclampsia than in term. Some genes had significant dysregulation both at term and preterm (e.g. *LGALS13, LGALS14),* while others only at preterm (e.g. *CSH1*). These data also reflect to the heterogeneous placental pathology behind the pathogenesis of preeclampsia, and the more severe pathologies in preterm.

Figure 4A-UU show gene comparisons for *ARNT2; BCL3; BCL6; BTG2; CDKN1A;* CGB3; *CLC; CLDN1; CRH; CSH1; CYP19A1; DUSP1; ENG; ERVFRDE1; ERVVVE1; ESRRG; FBLN1; FLT1; GATA2; GCM1; GH2; HLF; HSD1182; HSD1781; IKBKB; INSL4; JUNB; KIT; LEP; LGALS13; LGALS14; LGALS16, LGALS17A; MAPK13; NANOG; PAPPA; PAPPA2; PGF; PLAC1; POU5F1; SIGLEC6; TEAD3; TFAM, TFAP2A; TPBG; VDR;* and ZNF554 respectively.

**IV. Transcriptomic biomarkers**

The results of the neural network based analysis was a set of combinations of 2 to 8 genes, as assessed by the number of times they were retained as best predictors of blood pressure and birth-weight percentile when using different subsets of the training samples (Table 4). From these sets of combinations, six genes *(FLT1, HSD17B1, LEP, LGALS14, PAPPA2,* and *PLAC1)* were selected as being top 2 best predictors and / or highly placenta specific genes.

Table 4

| Best 2 predictors | X times out of 100 training-test sessions in top 5% |
|---|---|
| *HSD17B1 / PAPPA2* | 52 |
| *HSD17B1 / LEP* | 35 |
| *LEP / LGALS13* | 32 |
| *LGALS14 / PAPPA2* | 30 |
| *LEP / LGALS14* | 26 |

(continued)

| Best 2 predictors | X times out of 100 training-test sessions in top 5% |
|---|---|
| FLT1 / HSD17B1 | 25 |
| ENG / LGALS13 | 23 |
| CRH / LGALS14 | 21 |
| CSH1 / PAPPA2 | 21 |
| FLT1 / LGALS14 | 19 |
| **Best 3 predictors** | |
| HSD17B1 / KIT / PAPPA2 | 38 |
| CRH / HSD17B1 / PAPPA2 | 35 |
| CSH1 / HSD17B1 / PAPPA2 | 35 |
| HSD17B1 / LGALS13 / PAPPA2 | 35 |
| CLC / HSD17B1 / PAPPA2 | 33 |
| CLDN1 / HSD17B1 / PAPPA2 | 33 |
| FBLN1 / HSD17B1 / PAPPA2 | 33 |
| CGB3 / HSD17B1 / PAPPA2 | 32 |
| HSD17B1 / PAPPA2 / PLAC1 | 31 |
| CSH1 / LEP / SIGLEC6 | 30 |
| **Best 4 predictors** | |
| FBLN1 / HSD17B1 / LEP / SIGLEC6 | 34 |
| CRH / HSD1B2 / HSD17B1 / LGALS14 | 33 |
| CLC / HSD17B1 / KIT / PAPPA2 | 31 |
| CLC / HSD17B1 / LGALS13 / PAPPA2 | 31 |
| CRH / HSD17B1 / LGALS14 / PAPPA2 | 29 |
| HSD17B1 / LEP / LGALS13 / PAPPA2 | 28 |
| CRH / LGALS14 / PAPPA2 / TPBG | 26 |
| CLC / CRH / LGALS14 / PAPPA2 | 25 |
| CRH / HSD17B1 / KIT / PAPPA2 | 25 |
| CRH / HSD17B1 / LEP / LGALS14 | 25 |
| **Best 5 predictors** | |
| HSD17B1 / LEP / LGALS13 / PAPPA2 / SIGLEC6 | 32 |
| CRH / HSD1B2 / HSD17B1 / LGALS14 / PAPPA2 | 31 |
| CLC / CRH / KIT / LGALS14 / PAPPA2 | 30 |
| CSH1 / FLT1 / HSD17B1 / LEP / SIGLEC6 | 29 |
| HSD1B2 / HSD17B1 / LEP / LGALS13 / SIGLEC6 | 29 |
| CRH / CSH1 / ENG / LGALS14 / PAPPA2 | 28 |
| CRH / CSH1 / LGALS14 / PAPPA2 / TPBG | 28 |
| CRH / HSD11B2 / HSD17B1 / LGALS13 / TPBG | 28 |
| FBLN1 / LEP / LGALS14 / PAPPA2 / SIGLEC6 | 28 |

(continued)

| Best 5 predictors | |
|---|---|
| FLT1 / HSD1B2 / LEP / LGALS13 / SIGLEC6 | 28 |
| **Best 6 predictors** | |
| CRH / HSD11B2 / HSD17B1 / LGALS13 / LGALS14 / TPBG | 37 |
| CRH / CSH1 / HSD1B2 / HSD17B1 / LGALS14 / PAPPA2 | 33 |
| CLC / CRH / CSH1 / KIT / LGALS14 / PAPPA2 | 31 |
| HSD1B2 / KIT / LEP / LGALS14 / SIGLEC6 / TPBG | 30 |
| CRH / CSH1 / HSD11B2 / LGALS14 / PAPPA2 / TPBG | 29 |
| CSH1 / FBLN1 / HSD17B1 / LEP / PAPPA2 / SIGLEC6 | 29 |
| CLC / CRH / CSH1 / FBLN1 / LGALS14 / PAPPA2 | 28 |
| CRH / CSH1 / HSD11B2 / HSD17B1 / LGALS14 / SIGLEC6 | 28 |
| CRH / HSD1B2 / HSD17B1 / KIT / LGALS14 / PAPPA2 | 28 |
| ENG / FBLN1 / HSD17B1 / LEP / PAPPA2 / SIGLEC6 | 28 |
| **Best 7 predictors** | |
| CRH / HSD11B2 / HSD17B1 / LGALS13 / LGALS14 / PAPPA21 / TPBG | 40 |
| CRH / CSH1 / HSD1B2 / HSD17B1 / LGALS13 / LGALS14 / PAPPA2 | 36 |
| CRH / HSD11B2 / HSD17B1 / KIT / LGALS13 / LGALS14 / TPBG | 34 |
| CGB3 / CRH / HSD1B2 / HSD17B1 / LGALS13 / LGALS14 / TPBG | 31 |
| CRH / CSH1 / FLT1 / HSD1B2 / HSD17B1 / LGALS14 / PAPPA2 | 31 |
| CLC / CRH / CSH1 / FBLN1 / LGALS14 / PAPPA2 / PLAC1 | 30 |
| CRH / CSH1 / HSD11B2 / HSD17B1 / LGALS14 / PAPPA2 / PLAC1 | 30 |
| CRH / CSH1 / HSD11B2 / LGALS13 / LGALS14 / PAPPA2 / TPBG | 30 |
| CRH / CSH1 / LGALS13 / LGALS14 / PAPPA2 / PLAC1 / TPBG | 30 |
| CRH / CSH1 / ENG / HSD11B2 / HSD17B1 / LGALS14 / PAPPA2 | 29 |
| **Best 8 predictors** | |
| CRH / CSH1 / HSD11B2 / HSD17B1 / LGALS13 / LGALS14 / PAPPA2 / TPBG | 47 |
| CRH / CSH1 / FLT1 / HSD1B2 / HSD17B1 / LGALS13 / LGALS14 / TPBG | 37 |
| CGB3 / CRH / HSD11B2 / HSD17B1 / LGALS13 / LGALS14 / PAPPA2 / TPBG | 34 |
| CLC / CRH / HSD11B2 / HSD17B1 / KIT / LGALS13 / LGALSl4 / TPBG | 33 |
| CRH / FLT1 / HSD11B2 / HSD17B1 / LGALS13 / LGALS14 / PAPPA2 / TPBG | 33 |
| CLC / CRH / CSH1 / FBLN1 / FLT1 / KIT / LGALS14 / PAPPA2 | 32 |
| CRH / CSH1 / HSD11B2 / HSD17B1 / LGALS13 / LGALS14 / SIGLEC6 / TPBG | 32 |
| CRH / CSH1 / FBLN1 / HSD11B2 / HSD17B1 / LGALS13 / LGALS14 / TPBG | 31 |

(continued)

| Best 8 predictors | |
|---|---|
| CRH / CSH1 / FLT1 / HSD1B2 / HSD17B1 / LGALS13 / LGALS14 / PAPPA2 | 31 |
| CLC / CLDN1 / CRH / FBLN1 / HSD11B2 / HSD17B1 / KIT / LGALS14 | 30 |

The Linear Discriminant Analysis showed that the average sensitivity and specificity of the selected set of transcriptomic biomarkers for the detection of preeclampsia was 91.5% and 75%, respectively.

**V. Differentially expressed proteins in maternal serum in preeclampsia**

In the discovery phase, in the comparison of samples taken from healthy pregnant women with normal pregnancy outcome and those who subsequently developed preterm severe preeclampsia, 2080-2460 protein spots were identified on the gels. There were 39 protein spots, which were differentially expressed (29 down-regulated and 10 up-regulated) in at least 3 out of the 5 disease samples. The biggest difference in disease samples was 3.1-fold up-regulation and 3.1-fold down-regulation.

In the comparison of samples taken from healthy pregnant women with normal pregnancy outcome and those who subsequently developed term severe preeclampsia, 2130-2380 protein spots were identified on the gels. There were 20 protein spots, which were differentially expressed (11 down-regulated and 9 up-regulated) in at least 3 out of the 5 disease samples. The biggest difference in disease samples was a 3.9-fold up-regulation and a 4.5-fold down-regulation.

In the preparative phase, in the comparison of samples taken from healthy pregnant women with normal pregnancy outcome and those who subsequently developed preterm severe preeclampsia, there were ~2380 protein spots identified on the gels. From the 39 previously identified differentially expressed spots in preterm preeclampsia, 29 (25 down-regulated and 4 up-regulated) was identified and excised from the gels.

In the comparison of samples taken from healthy pregnant women with normal pregnancy outcome and those who subsequently developed term severe preeclampsia, there were ~2350 protein spots identified on the gels. From the 20 previously identified differentially expressed spots, 18 (11 down-regulated and 7 up-regulated) was identified and excised from the gels.

In the identification phase, the following differentially expressed proteins could be identified:

**A) Preterm preeclampsia**

| No | Direction | Gene symbol | ID | Protein Name |
|---|---|---|---|---|
| 1 | DOWNinPE | A1BG | gi\|21071030 | alpha 1B-glycoprotein precursor |
| 2 | DOWNinPE | AGT | gi\|532198 | Angiotensinogen |
| 3 | DOWNinPE | APOA4 | gi\|178757 | apolipoprotein A-IV precursor |
| 4 | DOWNinPE | APOL1 | gi\|12408013 | apolipoprotein L-I |
| 5 | DOWNinPE | CP | gi\|4557485 | ceruloplasmin precursor |
| 6 | DOWNinPE | C1QB | gi\|399140 | complement C1q subcomponent subunit B precursor |
| 7 | DOWNinPE | C7 | gi\|45580688 | complement component 7 precursor |
| 8 | DOWNinPE | C4 | gi\|2347136 | complement component C4 |
| 9 | DOWNinPE | CFB | gi\|291922 | complement factor B |
| 10 | DOWNinPE | CFH | gi\|148745112 | complement factor H |
| 11 | DOWNinPE | GSN | gi\|4504165 | gelsolin isoform a precursor |
| 12 | DOWNinPE | HPX | gi\|386789 | hemopexin precursor |
| 13 | DOWNinPE | HRG | gi\|4504489 | histidine-rich glycoprotein precursor |

(continued)

| No | Direction | Gene symbol | ID | Protein Name |
|---|---|---|---|---|
| 14 | DOWNinPE | IGFALS | gi\|4826772 | insulin-like growth factor binding protein, acid labile subunit |
| 15 | DOWNinPE | KNG1 | gi\|37748641 | kininogen 1 |
| 16 | DOWNinPE | PLG | gi\|387026 | Plasminogen |
| 17 | DOWNinPE | PAEP | gi\|182093 | pregnancy -as soci ated endometrial alpha2-globulin |
| 18 | DOWNinPE | GC | gi\|139641 | vitamin D-binding protein precursor |
| 19 | UPinPE | APOH | gi\|153266841 | apolipoprotein H precursor |
| 20 | UPinPE | C4 | gi\|2347136 | complement component C4 |

**B) Term preeclampsia**

| No | Direction | Gene symbol | ID | Protein Name |
|---|---|---|---|---|
| 1 | DOWNinPE | SERPINA3 | gi\|177809 | alpha-1-antichymotrypsin |
| 2 | DOWNinPE | CP | gi\|4557485 | ceruloplasmin precursor |
| 3 | DOWNinPE | C7 | gi\|45580688 | complement component 7 precursor |
| 4 | DOWNinPE | CFB | gi\|291922 | complement factor B |
| 5 | DOWNinPE | GSN | gi\|4504165 | gelsolin isoform a precursor |
| 6 | DOWNinPE | HRNR | gi\|28557150 | hornerin |
| 7 | DOWNinPE | ITIH2 | gi\|55958063 | inter-alpha (globulin) inhibitor H2 |
| 8 | UPinPE | AGT | gi\|532198 | angiotensinogen |
| 9 | UPinPE | CFB | gi\|291922 | complement factor B |
| 10 | UPinPE | FETUB | gi\|49902016 | fetuin B (alpha-2 Heremans-Schmid glycoprotein) |
| 11 | UPinPE | GSN | gi\|4504165 | gelsolin isoform a precursor |
| 12 | UPinPE | ITIH4 | gi\|31542984 | inter-alpha (globulin) inhibitor H4 |
| 13 | UPinPE | CD14 | gi\|3983127 | monocyte antigen CD14 precursor |
| 14 | UPinPE | PEDF | gi\|189778 | pigment epithelial-differentiating factor |
| 15 | UPinPE | PLG | gi\|387026 | plasminogen |
| 16 | UPinPE | GC | gi1139641 | vitamin D-binding protein precursor |

In each two comparisons, those candidates were selected which were differentially expressed in all disease specimens, had the highest fold-change, and the strongest p-value: complement factor B, gelsolin isoform a precursor, hornerin, fetuin B, hemopexin precursor, and apolipoprotein H precursor.

**Claims**

1. A method for assessing, in the first trimester of a pregnancy, the risk of developing preterm severe preeclampsia before the 36th week in a woman, comprising:
determining the level of apolipoprotein H precursor, having Accession Number gi\|153266841, in a blood sample obtained from the woman, wherein an increased level apolipoprotein H precursor is indicative of an increased risk of developing preterm severe preeclampsia before the 36th week.

2. Use of a kit in a method according to claim 1 comprising: detection mechanisms for determining the level of apolipoprotein H precursor in a blood sample obtained from the woman; instructions how to (i) generate a dataset based on the determined level(s), (ii) assess the risk of developing preeclampsia in the woman and (iii) determine a treatment regimen based on the assessed risk.

**Patentansprüche**

1. Ein Verfahren zur Beurteilung des Risikos der Entstehung einer vorzeitigen schweren Präeklampsie bei einer Frau vor der 36. Woche, umfassend die Bestimmung des Spiegels des Apolipoprotein H Präkursors mit der Akzessionsnummer gi 1153266841 in einer Frau entnommenen Blutprobe, in der ein erhöhter Spiegel an Apolipoprotein H Präkursor auf ein erhöhtes Risiko für die Entstehung einer vorzeitigen schweren Präeklampsie vor der 36. Woche hinweist.

2. Verwendung eines Kits in einem Verfahren nach Anspruch 1, umfassend: Nachweistechniken zur Bestimmung des Spiegels von Apolipoprotein H Präkursor in einer Frau entnommenen Blutprobe; Anweisungen, wie (i) ein Datensatz basierend auf den ermittelten Werten zu erstellen ist; (ii) das Risiko der Entstehung einer Präeklampsie in einer Frau zu beurteilen ist und (iii) ein Behandlungsregime auf Basis des eingeschätzten Risikos festzulegen ist.

**Revendications**

1. Procédé d'évaluation, dans le premier trimestre d'une grossesse, du risque de développer une prééclampsie sévère avant terme avant la 36ème semaine dans une femme, comprenant :
la détermination du niveau de précurseur d'apolipoprotéine H, ayant un numéro d'accès gi|153266841, dans un échantillon de sang obtenu à partir de la femme, où un précurseur d'apolipoprotéine H de niveau accru est indicative d'un risque accru de développer une pré-éclampsie sévère avant terme avant la 36ème semaine.

2. Utilisation d'un kit dans un procédé selon la revendication 1 comprenant : des mécanismes de détection pour déterminer le niveau de précurseur d'apolipoprotéine H dans un échantillon de sang obtenu à partir de la femme; des instructions comment (i) générer un ensemble de données sur la base du ou des niveaux déterminés, (ii) évaluer le risque de développer une prééclampsie dans la femme et (iii) déterminer un régime de traitement sur la base du risque évalué.

Figure 1

Figure 2A

Figure 2B

Figure 2C

Figure 3

ARNT2

Figure 4A

BCL3

Figure 4B

BCL6

Figure 4C

BTG2

Figure 4D

CDKN1A

Figure 4E

CGB3

Figure 4F

CLC

Figure 4G

CLDN1

Figure 4H

## CRH

Figure 4I

## CSH1

Figure 4J

## CYP19A1

Figure 4K

## DUSP1

Figure 4L

**ENG**

Figure 4M

**ERVFRDE1**

Figure 4N

**ERVWE1**

Figure 4O

**ESRRG**

Figure 4P

**FBLN1**

Figure 4Q

**FLT1**

Figure 4R

**GATA2**

Figure 4S

**GCM1**

Figure 4T

## GH2

Figure 4U

## HLF

Figure 4V

## HSD11B2

Figure 4W

## HSD17B1

Figure 4X

IKBKB

Figure 4Y

INSL4

Figure 4Z

JUNB

Figure 4AA

KIT

Figure 4BB

**LEP**

Figure 4CC

**LGALS13**

Figure 4DD

**LGALS14**

Figure 4EE

**LGALS16**

Figure 4FF

**LGALS17A**

Figure 4GG

**MAPK13**

Figure 4HH

**NANOG**

Figure 4II

**PAPPA**

Figure 4JJ

PAPPA2

Figure 4KK

PGF

Figure 4LL

PLAC1

Figure 4MM

POU5F1

Figure 4NN

## SIGLEC6

Figure 4OO

## TEAD3

Figure 4PP

## TFAM

Figure 4QQ

## TFAP2A

Figure 4RR

**TPBG**

Figure 4SS

**VDR**

Figure 4TT

**ZNF554**

Figure 4UU

## REFERENCES CITED IN THE DESCRIPTION

**Patent documents cited in the description**

- US 5143854 A
- US 6087112 A
- US 5215882 A
- US 5707807 A
- US 5807522 A
- US 5958342 A
- US 5994076 A
- US 6004755 A
- US 6048695 A
- US 6060240 A
- US 6090556 A
- US 6040138 A

**Non-patent literature cited in the description**

- **JANSSEN, B.G. et al.** *Particle and Fibre Toxicology,* 2013, vol. 10, 22
- **TABISH, A.M. et al.** *PLoS ONE,* 2012, vol. 7, e34674
- **GODDERIS, L. et al.** *Epigenomics,* 2012, vol. 4, 269-277
- **PROCTOR, M. et al.** *Clin. Chem.,* 2006, vol. 52, 1276-1283
- **SHEN, L. et al.** *Curr. Opin. Clin. Nutr. Metab. Care.,* 2007, vol. 10, 576-81
- **GU H et al.** *Nature Methods,* 2010, vol. 7, 133-138
- **BOCK C et al.** *Nature Biotech.,* 2010, vol. 28, 1106-1114
- **HARRIS RA et al.** *Nature Biotech.,* 2010, vol. 28, 1097-1105
- **EISEN, M.** SCANALYZE User Manual. Stanford Univ, 1999
- **SIBAI, B et al.** Pre-eclampsia. *Lancet,* 2005, vol. 365, 785-99
- **SU, AI et al.** A gene atlas of the mouse and human protein-encoding transcriptomes. *PNAS,* 2004, vol. 101, 6062-67
- **TARCA, A.L. ; THAN, N.G. ; ROMERO, R.** Methodological Approach from the Best Overall Team in the IMPROVER Diagnostic Signature Challenge. *Systems Biomedicine submitted,* 2013
- *In-Gel Digest Procedure, msfacility.ucsf.edu/in-gel.html*
- **ROSENFELD et al.** *Anal. Biochem.,* 1992, vol. 203 (1), 173-179
- **HELLMAN et al.** *Anal. Biochem.,* 1995, vol. 224 (1), 451-455